# EUROPEAN PATENT APPLICATION

(11) **EP 2 298 358 A1**
(43) Date of publication of application: **23.03.2011**
(21) Application number: 10182758.2
(22) Date of filing: 06.05.2003
(51) Int. Cl.: A61K 48/00, C12N 15/00, C12N 15/63

(54) **Methods for delivery of nucleic acids**

(30) Priority: 06.05.2002 US 378191 P
(62) Divisional of application: 03747676.9
(71) Applicant: Alnylam Pharmaceuticals Inc., Cambridge MA 02142 (US)
(72) Inventor: Satishchandran, C., Landsdale, PA 19446 (US)
(74) Representative: HOFFMANN EITLE

(57) **Abstract**

This invention features methods and compositions for delivery of nucleic acids (e.g. DNA, RMA, PNA, and hybrids thereof) to cells. The nucleic acid delivery complexes of the invention permit biologically active nucleic acids to be delivered to cells and organisms *in vitro* and *in vivo* in a manner and form that allows the nucleic acids to carry out their desired biological function.

## Description

### Cross-Reference to Related Application

This application claims the benefit of U.S. provisional application 60/378,191, filed May 6, 2002.

### Background of the Invention

This invention relates to methods and compositions for delivery of nucleic acids (e.g., DNA, RNA, hybrid, heteroduplex, and modified nucleic acids) to cells. The nucleic acid delivery complexes of the invention permit biologically active nucleic acids to be delivered to cells and organisms *in vitro* and *in vivo* in a manner and form that allows the nucleic acids to carry out their desired biological function.

Nucleic acids (e.g., DNA, RNA, hybrid, heteroduplex, and modified nucleic acids) have come to be recognized as extremely valuable agents with significant and varied biological activities, including their use as therapeutic moieties in the prevention and/or treatment of disease states in man and animals. For example, oligonucleotides acting through antisense mechanisms are designed to hybridize to target mRNAs, thereby modulating the activity of the mRNA. Another approach to the utilization of nucleic acids as therapeutics is designed to take advantage of triplex or triple strand formation, in which a single-stranded oligomer (e.g., DNA or RNA) is designed to bind to a double-stranded DNA target to produce a desired result, e.g., inhibition of transcription from the DNA target. Yet another approach to the utilization of nucleic acids as therapeutics is designed to take advantage of ribozymes, in which a structured RNA or a modified oligomer is designed to bind to an RNA or a double-stranded DNA target to produce a desired result, e.g., targeted cleavage af RNA or the DNA target and thus inhibiting its expression. Nucleic acids may also be used as immunizing agents, e.g., by introducing DNA molecules into the tissues or cells of an organism that express proteins capable of eliciting an immune response. Nucleic acids may also be engineered to encode an RNA with antisense, ribozyme, or triplex activities, or to produce RNA that is translated to produce protein(s) that have biological function. More recently, the phenomenon of RNAi or double-stranded RNA (dsRNA)-mediated gene silencing has been recognized, whereby dsRNA complementary to a region of a target gene in a cell or organism inhibits expression of the target gene (see, e.g., WO 99/32619, published 1 July 1999, Fire et al.*;* and U.S. 6,506,559: "Genetic Inhibition by Double-Stranded RNA;" WO 00/63364: "Methods and Compositions for Inhibiting the Function of Polynucleotide Sequences," Pachuk and Satishchandran; and U.S.S.N. 60/419,532, filed October 18, 2002).

Whatever the intended mechanism of biological activity, successful utilization of nucleic acids as therapeutic moieties depends upon an ability to deliver the selected nucleic acid to the target host cell in a therapeutically relevant manner, e.g., in a biologically active, non-toxic form to the desired cell or cells *in vivo* or *in vitro,* in the desired cytosolic location of a target cell.

It is possible to transfer genetic material into target cells without the use of vectors or carriers. For example, DNA injected by itself into various tissues is able to enter cells and express a protein that elicits an immune response. While such "naked DNA" can be taken up by cells and express encoded proteins (U.S.5,589,466, Felgner et al.), efforts to transfect naked plasmid DNA tend to yield variable results, lacking in reproducibility and predictability. This result is likely due to the fact that DNA is negatively charged and *in vivo* binds proteins and other molecules having cationic side chains. In addition, DNA by itself is hydrophilic, and the hydrophobic character of the cellular membrane poses a significant barrier to the transfer of DNA across it.

Delivery of genes can be achieved through the use of viral vectors. However, viral vectors may induce immune responses to the vehicle itself and undesired host responses. Non-viral gene delivery is therefore a desirable approach to deliver genes. Non-viral vectors are also predicted to be more stable than viral vectors.

Accordingly, DNA and other nucleic acids are more frequently transfected into cells complexed with cationic lipids as well as a variety of other molecules. Although cationic lipid based complexing agents have predominated in the cellular transfection arena, cationic lipid-based transfection has not demonstrated a rational and predictable correlation between structure and function, particularly for *in vivo* applications. While numerous transfection technologies have been developed, only a few have shown promise for *in vivo* applications. Lacking a clear scientific framework, it has been necessary to resort to empirical methods to deliver plasmid DNA by different delivery routes and through the use of diverse technical approaches.

Yet despite the availability of these and other agents, there remains a significant need for improved delivery of nucleic acids to cells, especially for *in vivo* use and such new therapeutic applications as RNAi.

### Summary Of The Invention

In one aspect, the invention features a composition that includes a nucleic acid, an endosomolytic spermine that includes a cholesterol or fatty acid, and a targeting spermine that includes a ligand for a cell surface molecule. The ratio of positive to negative charge of the composition is between 0.5 and 1.5, inclusive; the endosomolytic spermine constitutes at least 20% of the spermine-containidg molecules in the composition; and the targeting spermine constitutes at least 10% of the spermine-containing molecules in the composition. Desirably, the ratio of positive to negative charge is between 0.8 and 1.2, inclusive, such as between 0.8 and 0.9, inclusive.

When the nucleic acid includes DNA, the endosomolytic spermine desirably constitutes between 40% and 90%, inclusive, of the spermine-containing molecules in the composition, and/or the targeting spermine constitutes between 10% and 60%, inclusive, of the spermine-containing molecules in the composition. When the nucleic acid is RNA, the endosomolytic spermine constitutes between 20% and 90%, inclusive, of the spermine-containing molecules in the composition, and/or the targeting spermine constitutes between 10% and 80%, inclusive, of the spermine-containing molecules in the composition. In desirable embodiments, the targeting spermine constitutes between 30 and 40%, inclusive, of the spermine-containing molecules in the composition, and the endosomolytic spermine constitutes between 60 and 70%, inclusive of the spermine-containing molecules in the composition. Desirably, the targeting spermine constitutes 35% of the spermine-containing molecules in the composition, and the endosomolytic spermine constitutes 65% of the spermine-containing molecules in the composition.
In some embodiments, the composition further includes a spermine-containing molecule that does not contain a cholesterol, a fatty acid, or a ligand for a cell surface molecule. This additional spermine-containing molecule may be an unmodified or modified spermine (e.g., spermine modified with one or more branched or unbranched PEG linkers to increase bioavailability).

Desirably, the ionic strength of the composition is equivalent to the ionic strength of a solution containing between 50 mM and 240 mM sodium, inclusive, such as between 125 mM and 175 mM sodium, inclusive, (e.g., 150 mM sodium). In desirable embodiments, the pH of the composition is between 6 and 8, inclusive, such as between 6 and 7, inclusive, or between 6.5 and 6.8, inclusive.

In various embodiments, the nucleic acid is a DNA, RNA, DNAIRNA hybrid, or peptide nucleic acid. The nucleic acid may be, e.g., linear, circular, or supercoiled. The nucleic acid may also be single stranded or double stranded. In various embodiments, the nucleic acid is less than 45, 40, 30, 25, 10, 5, or 1 kilobase in length. In some embodiments, the length of the nucleic acid is less than 500, 100, 50, or 25 bases in length. Desirably, the composition includes between 1 and 50 µg of nucleic acid, such as between 1 and 30 µg or 10 and 20 µg.

In some embodiments, one endosomolytic spermine includes two cholesterols, two fatty acids, or one cholesterol and one fatty acid. The fatty acids may be the same or different. In various embodiments, the targeting spermine includes two ligands for a cell surface molecule. The ligands may be the same or different. In desirable embodiments, composition includes at least two different endosomolytic spermines and/or at least two different targeting spermines. Desirably, the use of two or more targeting spermines increases the specificity of the composition for a particular cell type by at least 25, 50, 75, 100, 200, 500, or 700%. In some embodiments, one endosomolytic spermine includes a cholesterol and one endosomolytic spermine includes a fatty acid. Desirably, such a composition includes more cholesterol moieties than fatty acid moieties. In various embodiments, the ligand is a peptide (e.g., a peptide of less than 100, 50, or 10 amino acids or a peptide with an RGD motif), antibody, biotin, folate receptor ligand, lactose, fucose (e.g., a ligand for a fucose receptor on M-cells or carcinoma cells), or mannose moiety. Desirably, the ligand is bound to a secondary amine in a spermine through a linker and/or an oxygen at the C3 position in the cholesterol is bound to a secondary amine in a spermine through a linker. Desirably, the fatty acid is bonded directly to a secondary amine in a spermine and has a free carboxylic acid group (COOH). Exemplary linkers contain between 3 and 12 carbon atoms, inclusive, such as a saturated or unsaturated C₃ to C₁₂ hydrocarbon moiety, inclusive. In desirable embodiments, the linker contains 3 or 4 carbon atoms and no double bonds, the linker contains 5 or 6 carbon atoms and at most 1 double bond, or the linker contains between 7 or 12 carbon atoms, inclusive, and at most 2 double bonds. Desirably, the linker contains 5 carbon atoms. In desirable embodiments, the linker is an unbranched alkyl group. In some embodiments, the linker is a branched or unbranched PEG. Desirably, the linker is bound through a terminal carboxyl, amino, hydroxyl, sulfhydryl, alkyl, carboxamide, carbamate, thiocarbamate, or carbamoyl bridging group to a secondary amine group of the spermine.

Desirably, the fatty acid contains between 4 or 12 carbon atoms, inclusive. In various embodiments, the linker is a saturated or unsaturated C₄ to C₁₂ hydrocarbon moiety, inclusive, that is desirably unbranched. In some embodiments, the fatty acid includes an ester group, and/or contains 6 carbon atoms. The pKa of the carboxyl group of the fatty acid is desirably at most 6.

In a related aspect, the invention features a pharmaceutical composition that includes a composition of the invention (e.g., any composition of the above aspect) and a pharmaceutically acceptable carrier. Desirably, the pH of the composition is between 5 and 8, inclusive, such as between 6 and 7.5, inclusive. Desirably, the composition is isotonic relative to the electrolyte concentration of human blood. Desirably, the composition includes between 1 and 50 µg of nucleic acid, such as between 1 and 30 µg or 10 and 20 µg.

In another aspect, the invention features a composition that includes a nucleic acid complexed with a cationic amphiphile in an oil-in-water emulsion (e.g., an emulsion with over 50% water) in which at least 10% of the complex is in the oil phase of the emulsion. Desirably, at least 25, 50, 50, 70, 75, 80, 90, 95, 98, or 99% of the nucleic acid is in the oil phase of the emulsion. In various embodiments, the cationic amphiphile is a cationic lipid, modified or unmodified spermine (e.g., spermine modified with a hydrophobic group such as a fatty acid, including a C₃ to C₂₀ fatty acid, cholesterol, a fatty acid and a cholesterol, two fatty acids, or two cholesterols), bupivacaine, or benzalkonium chloride. In certain embodiments, the cationic amphiphile is (i) bupivacaine and the ratio of positive to negative charge is between 1 and 5, inclusive; (ii) unmodified or modified spermine and the ratio of positive to negative charge is between 0.5 to 1.5, inclusive; (iii) a cationic lipid and the ratio of positive to negative charge is between 0.5 and 5.0, inclusive, (iv) lipofectarninc and the ratio of positive to negative charge is between 0.5 and 2.0, inclusive; or (v) benzalkonium chloride and the ratio of positive to negative charge is between is 0.01 and 0.2, inclusive. Desirably, the oil is a vegetable (e.g., soybean or corn oil) or animal oil, such as an oil approved for human consumption.
In desirable embodiments, the pH of the composition is between 6 and 8, inclusive, such as between 6 and 7, inclusive, or between 6.5 and 6.8, inclusive.

In another aspect, the invention features a composition that includes a double stranded RNA and a local anesthetic, e.g., bupivacaine. In desirable embodiments, the pH of the composition is between 6 and 8, inclusive, such as between 6 and 7, inclusive, or between 6.5 and 6.8, inclusive.

In various embodiments of any of the above aspects, the nucleic acid is a DNA, RNA, DNA/RNA hybrid, or peptide nucleic acid. The nucleic acid may be, e.g., linear, circular, or supercoiled. The nucleic acid may also be single stranded or double stranded. In various embodiments, the nucleic acid is less than 45, 40, 30, 25, 10, 5, or 1 kilobase in length. In some embodiments, the length of the nucleic acid is less than 500, 100, 50, or 25 bases in length. Desirably, the composition includes between 1 and 50 µg of nucleic acid, such as between 1 and 30 µg or 10 and 20 µg.

In a related aspect, the invention features a pharmaceutical composition that includes a composition of the invention (e.g., any composition of any of the above aspects) and a pharmaceutically acceptable carrier. Desirably, the pH of the composition is between 5 and 8, inclusive, such as between 6 and 7.5, inclusive. Desirably, the composition is isotonic relative to the electrolyte concentration of human blood. Desirably, the composition includes between 1 and 50 µg of nucleic acid, such as between 1 and 30 µg or 10 and 20 µg.

The above compositions of the invention are useful in a variety of applications for either expressing an RNA or protein of interest (e.g., gene therapy or DNA vaccines) or for silencing an RNA or protein of interest (e.g., ribozymes, RNAi, or antisense).

In one such aspect, the invention features a method for expressing an RNA or protein molecule of interest in a cell. The method includes contacting a cell with a composition of the invention under conditions that desirably allow introduction of a nucleic acid into the cell and expression of an RNA or protein of interest encoded by a nucleic acid in the composition. In some embodiments, the cell has a mutation associated with a disease or disorder in an endogenous form of the RNA or protein of interest, and the nucleic acid encodes a form of the RNA or protein that is not associated with the disease or disorder. In certain embodiments, the RNA or protein of interest is from a pathogen, and the method causes an immune response against the RNA or protein of interest.

In another aspect, the invention features a method for inhibiting the expression of a target nucleic acid in a cell. The method includes contacting a cell with a composition of the invention under conditions that desirably allow introduction of a nucleic acid into the cell and expression of a ribozyme encoded by a nucleic acid in the composition. The ribozyme cleaves a target nucleic acid in the cell that is associated with a disease, disorder, or infection.

In a related aspect, the invention features another method for inhibiting the expression of a target nucleic acid in a cell. The method includes contacting a cell with a composition of the invention under conditions that desirably allow introduction of a nucleic acid into the cell. The composition includes a first double stranded RNA (dsRNA) or a nucleic acid encoding a first double stranded dsRNA that has substantial sequence identity to a region of the target nucleic acid and specifically inhibits expression of the target nucleic acid. Desirably, the method further includes introducing a short, second dsRNA or a nucleic acid encoding a short, second dsRNA that inhibits dsRNA-mediated toxicity into the cell.

In another aspect, the invention provides a method for treating, stabilizing, or preventing a disease, disorder, or infection in an animal. The method includes contacting an animal with a composition of the invention under conditions that desirably allow introduction of a nucleic acid into the animal. The composition includes a first dsRNA or a nucleic acid encoding a first double stranded dsRNA that has substantial sequence identity to a region of the target nucleic acid associated with the disease, disorder, or infection and specifically inhibits expression of the target nucleic acid. Desirably, the method further includes introducing a short, second dsRNA or a nucleic acid encoding a short, second dsRNA that inhibits dsRNA-mediated toxicity into the cell.

In another aspect, the invention features another method for inhibiting the expression of a target nucleic acid in a cell. The method that includes contacting a cell with a composition of the invention under conditions that desirably allow introduction of a nucleic acid into the cell. The composition includes an antisense nucleic acid that has substantial sequence identity to a region of the target nucleic acid and specifically inhibits expression of the target nucleic acid.

In yet another aspect, the invention features a method for treating, stabilizing, or preventing a disease, disorder, or infection in an animal. The method includes contacting an animal with a composition of the invention under conditions that desirably allow introduction of a nucleic acid into the animal. The composition includes an antisense nucleic acid that has substantial sequence identity to a region of the target nucleic acid associated with the disease, disorder, or infection and specifically inhibits expression of the target nucleic acid. In some embodiments, the target nucleic acid is associated with a pathogen, such as a virus, bacterium, yeast, or infectious agent.

Another aspect of the invention includes a method for identifying a nucleic acid that modulates a detectable phenotype in a cell. The method includes contacting or transforming a population of cells with a composition of the invention that includes a library of nucleic acids (e.g., 2, 5, 10, 100, 1000, 5000, 10000 or more different nucleic acids), wherein at least two cells of the population of cells are each transformed with a different nucleic acid from the library, and assaying for a modulation in the detectable phenotype. The modulation identifies a nucleic acid that is associated with the phenotype. Desirably, the library includes a first dsRNA molecule or a nucleic acid that encodes a first dsRNA molecule. Desirably, the method further includes introducing a short, second dsRNA or a nucleic acid encoding a short, second dsRNA that inhibits dsRNA-mediated toxicity into the cell. In desirable embodiments, the library includes antisense nucleic acids or nucleic acids that encode antisense nucleic acids. In various embodiments, the modulation in a detectable phenotype is a modulation in the function of a cell, a modulation in the biological activity of a polypeptide, or a modulation in the expression of a target nucleic acid. Desirably, the method further includes identifying the nucleic acid by amplifying the nucleic acid and sequencing the amplified nucleic acid. Desirably, the library includes cDNA molecules derived from the cells.

In various embodiments of any of the aspects of the invention, the double stranded region in the second dsRNA contains between 11 and 30 nucleotides, inclusive, and/or the double stranded region in the first dsRNA contains between 11 and 30 nucleotides, inclusive. In some embodiments, the double stranded region in the first dsRNA includes over 30, 50, 100, 200, 1000, or 2000 nucleotides. Desirably, the cell is a vertebrate cell, such as a mammalian cell (e.g., human cell). In desirable embodiments, the cell is in a mammal (e.g., a human). Desirably, the animal is mammal, such as a human.

In some embodiments of any of the aspects of the invention, the nucleic acid is a multiple epitope dsRNA or multiple epitope antisense nucleic acid. Desirably, the methods of the invention are conducted under conditions that inhibit or prevent an interferon response or a dsRNA stress response. In some embodiments, the spermine is modified with one or more branched or unbranched PEG linkers to increase bioavailability.

In other embodiments of any aspect of the invention, the nucleic acid includes one or more modified nucleotides in which the 2' position in the sugar contains a halogen (such as flourine group) or contains an alkoxy group (such as a methoxy group) which increases the half-life of the nucleic acid *in vitro* or *in vivo* compared to the corresponding nucleic acid in which the corresponding 2' position contains a hydrogen or an hydroxyl group. In yet other embodiments, the nucleic acid includes one or more linkages between adjacent nucleotides other than a naturally-occurring phosphodiester linkage. Examples of such linkages include phosphoramide, phosphorothioate, and phosphorodithioate linkages. Desirably, the composition is soluble in an isotonic solution. In other desirable embodiments, the size of the particles in the composition is less than 150, 100, or 50 nm so that there is minimal aggregation of the particles into insoluble structures.

Exemplary target nucleic acids to be silenced include nucleic acids associated with cancer or abnormal cell growth, such as oncogenes, and nucleic acids associated with an autosomal dominant or recessive disorder (see, for example, WO 00/63364, WO 00/44914, and WO 99/32619). Desirably, the dsRNA or antisense nucleic acid inhibits the expression of an allele of a nucleic acid that has a mutation associated with a dominant disorder and does not substantially inhibit the other allele of the nucleic acid (*e.g*, an allele without a mutation associated with the disorder). Other exemplary target nucleic acids to be silenced include host cellular nucleic acids or pathogen nucleic acids required for the infection or propagation of a pathogen, such as a virus, bacteria, yeast, protozoa, or parasite.

Other embodiments of the invention include embodiments described in U.S.S.N. 60/419,532, filed October 18, 2002.

### Definitions

By "nucleic acid composition" is meant any one or more compounds in which one or more molecules of phosphoric acid are combined with a carbohydrate (e.g., pentose or hexose) which are in turn combined with bases derived from purine (e.g., adenine) and from pyrimidine (e.g., thymine). Particular naturally occurring nucleic acid molecules include genomic deoxyribonucleic acid (DNA) and genomic ribonucleic acid (RNA), as well as the several different forms of the latter, e.g., messenger RNA (mRNA), transfer RNA (tRNA), and ribosomal RNA (rRNA). Also included are different DNA molecules which are complementary (cDNA) to the different RNA molecules. Synthesized DNA or a hybrid thereof with naturally occurring DNA, as well as DNA/RNA hybrids, and PNA molecules (Gambari, Curr Pharm Des 2001 Nov;7(17):1839-62) can also be used.

Nucleic acids typically have a sequence of two or more covalently bonded naturally-occurring or modified deoxyribonucleotides or ribonucleotides. Modified nucleic acids include, e.g., peptide nucleic acids and nucleotides with unnatural bases.

By "dsRNA" is meant a nucleic acid containing a region of two or more nucleotides that are in a double stranded conformation. In various embodiments, the dsRNA consists entirely of ribonucleotides or consists of a mixture of ribonucleotides and deoxynucleotides, such as the RNA/DNA hybrids disclosed, for example, by WO 00/63364, filed April 19, 2000 or U.S.S.N. 60/130,377, filed April 21, 1999. The dsRNA may be a single molecule with a region of self-complimentarity such that nucleotides in one segment of the molecule base pair with nucleotides in another segment of the molecule. In various embodiments, a dsRNA that consists of a single molecule consists entirely of ribonucleotides or includes a region of ribonucleotides that is complimentary to a region of deoxyribonucleotides. Alternatively, the dsRNA may include two different strands that have a region of complimentarity to each other. In various embodiments, both strands consist entirely of ribonucleotides, one strand consists entirely of ribonucleotides and one strand consists entirely of deoxyribonucleotides, or one or both strands contain a mixture of ribonucleotides and deoxyribonucleotides. Desirably, the regions of complimentarity are at least 70, 80, 90, 95, 98, or 100% complimentary. Desirably, the region of the dsRNA that is present in a double stranded conformation includes at least 5, 10, 20, 30, 50, 75,100, 200, 500, 1000, 2000 or 5000 nucleotides or includes all of the nucleotides in a cDNA being represented in the dsRNA. In some embodiments, the dsRNA does not contain any single stranded regions, such as single stranded ends, or the dsRNA is a hairpin. In other embodiments, the dsRNA has one or more single stranded regions or overhangs, Desirable RNA/DNA hybrids include a DNA strand or region that is an antisense strand or region (*e.g,* has at least 70, 80, 90, 95, 98, or 100% complimentary to a target nucleic acid) and an RNA strand or region that is an sense strand or region (*e.g,* has at least 70, 80, 90, 95, 98, or 100% identity to a target nucleic acid). In various embodiments, the RNA/DNA hybrid is made *in vitro* using enzymatic or chemical synthetic methods such as those described herein or those described in WO 00/63364, filed April 19, 2000 or U.S.S.N. 60/130,377, filed April 21, 1999. In other embodiments, a DNA strand synthesized *in vitro* is complexed with an RNA strand made *in vivo* or *in vitro* before, after, or concurrent with the transformation of the DNA strand into the cell. In yet other embodiments, the dsRNA is a single circular nucleic acid containing a sense and an antisense region, or the dsRNA includes a circular nucleic acid and either a second circular nucleic acid or a linear nucleic acid (see, for example, WO 00/63364, filed April 19, 2000 or U.S.S.N. 60/130,377, filed April 21, 1999.) Exemplary circular nucleic acids include lariat structures in which the free 5' phosphoryl group of a nucleotide becomes linked to the 2' hydroxyl group of another nucleotide in a loop back fashion.

In other embodiments, the dsRNA contains one or two capped strands or no capped strands, as disclosed, for example, by WO 00/63364, filed April 19, 2000 or U.S.S.N. 60/130,377, filed April 21, 1999. In other embodiments, the dsRNA contains coding sequence or non-coding sequence, for example, a regulatory sequence (*e.g.*, a transcription factor binding site, a promoter, or a 5' or 3' untranslated region (UTR) of an mRNA). Additionally, the dsRNA can be any of the at least partially double-stranded RNA molecules disclosed in WO 00/63364, filed April 19, 2000 (see, for example, pages 8-22). Any of the dsRNA molecules may be expressed *in vitro* or *in vivo* using the methods described herein or standard methods, such as those described in WO 00/63364, filed April 19, 2000 (see, for example, pages 16-22).

By "short dsRNA" is meant a dsRNA that has 45, 40, 35, 30, 27, 25, 23, 21, 18, 15, 13, or fewer contiguous nucleotides in length that are in a double stranded conformation. Desirably, the short dsRNA is at least 11 nucleotides in length. In desirable embodiments, the double stranded region is between 11 to 45, 11 to 40, 11 to 30, 11 to 20, 15 to 20, 15 to 18,20 to 25, 21 to 23, 25 to 30, or 30 to 40 contiguous nucleotides in length, inclusive. In some embodiments, the short dsRNA is between 30 to 50, 50 to 100, 100 to 200, 200 to 300, 400 to 500, 500 to 700, 700 to 1000, 1000 to 2000, or 2000 to 5000 nucleotides in length, inclusive and has a double stranded region that is between 11 and 40 contiguous nucleotides in length, inclusive. In one embodiment, the short dsRNA is completely double stranded. In some embodiments, the short dsRNA is between 11 and 30 nucleotides in length, and the entire dsRNA is double stranded. In other embodiments, the short dsRNA has one or two single stranded regions. In particular embodiments, the short dsRNA binds PKR or another protein in a dsRNA-mediated stress response pathway. Desirably, the short dsRNA inhibits the dimerization and activation of PKR by at least 20, 40, 60, 80, 90, or 100%. In some desirable embodiments, the short dsRNA inhibits the binding of a long dsRNA to PKR or another component of a dsRNA-mediated stress response pathway by at least 20, 40, 60, 80, 90, or 100%.

By "multiple epitope dsRNA" is meant an RNA molecule that has segments derived from multiple target nucleic acids or that has non-contiguous segments from the same target nucleic acid. For example, the multiple epitope dsRNA may have segments derived from (i) sequences representing multiple genes of a single organism; (ii) sequences representing one or more genes from a variety of different organisms; and/or (iii) sequences representing different regions of a particular gene (e.g., one or more sequences from a promoter and one or more sequences from a coding region such as an exon). Desirably, each segment has substantial sequence identity to the corresponding region of a target nucleic acid, In various desirable embodiments, a segment with substantial sequence identity to the target nucleic acid is at least 30, 40, 50, 100, 200, 500, 750, or more nucleotides in length, In desirable embodiments, the multiple epitope dsRNA inhibits the expression of at least 2, 4, 6, 8, 10, 15, 20, or more target genes by at least 20, 40, 60, 80, 90, 95, or 100%. In some embodiments, the multiple epitope dsRNA has non-contiguous segments from the same target gene that may or may not be in the naturally occurring 5' to 3' order of the segments, and the dsRNA inhibits the expression of the nucleic acid by at least 50, 100, 200, 500, or 1000% more than a dsRNA with only one of the segments.

By "antisense" is meant a nucleic acid, regardless of length, that is complementary to a coding strand or mRNA of interest. In some embodiments, the antisense molecule inhibits the expression of only one molecule of interest, and in other embodiments, the antisense molecule inhibits the expression of more than one molecule of interest. Desirably, the antisense nucleic acid decreases the expression or biological activity of an RNA or protein of interest by at least 20, 40, 50, 60, 70, 80, 90, 95, or 100%. A antisense molecule can be introduced, e.g., to an individual cell or to whole animals, for example, it may be introduced systemically via the bloodstream. Desirably, a region of the antisense nucleic acid or the entire antisense nucleic acid is at least 70, 80, 90, 95, 98, or 100% complimentary to a coding sequence, regulatory region (5' or 3' untranslated region), or an mRNA of interest. Desirably, the region of complementarity includes at least 5, 10, 20, 30, 50, 75,100, 200, 500, 1000, 2000 or 5000 nucleotides or includes all of the nucleotides in the antisense nucleic acid.

In some embodiments, the antisense molecule is less than 200,150, 100, 75, 50, or 25 nucleotides in length. In other embodiments, the antisense molecule is less than 50,000; 10,000; 5,000; or 2,000 nucleotides in length. In certain embodiments, the antisense molecule is at least 200, 300, 500, 1000, or 5000 nucleotides in length. In some embodiments, the number of nucleotides in the antisense molecule is contained in one of the following ranges: 5-15 nucleotides, 16-20 nucleotides, 21-25 nucleotides, 26-35 nucleotides, 36-45 nucleotides, 46-60 nucleotides, 61-80 nucleotides, 81-100 nucleotides, 101-150 nucleotides, or 151-200 nucleotides, inclusive. In addition, the antisense molecule may contain a sequence that is less than a full length sequence or may contain a full-length sequence.

By "treating, stabilizing, or preventing a disease or disorder" is meant preventing or delaying an initial or subsequent occurrence of a disease or disorder; increasing the disease-free survival time between the disappearance of a condition and its reoccurrence; stabilizing or reducing an adverse symptom associated with a condition; or inhibiting or stabilizing the progression of a condition. Desirably, at least 20,40, 60, 80, 90, or 95% of the treated subjects have a complete remission in which all evidence of the disease disappears. In another embodiment, the length of time a patient survives after being diagnosed with a condition and treated using a method of the invention is at least 20, 40, 60, 80, 100, 200, or even 500% greater than (i) the average amount of time an untreated patient survives or (ii) the average amount of time a patient treated with another therapy survives.

By "treating, stabilizing, or preventing cancer" is meant causing a reduction in the size of a tumor, slowing or preventing an increase in the size of a tumor, increasing the disease-free survival time between the disappearance of a tumor and its reappearance, preventing an initial or subsequent occurrence of a tumor, or reducing or stabilizing an adverse symptom associated with a tumor. In one embodiment, the percent of cancerous cells surviving the treatment is at least 20, 40, 60, 80, or 100% lower than the initial number of cancerous cells, as measured using any standard assay. Desirably, the decrease in the number of cancerous cells induced by administration of a composition of the invention is at least 2, 5, 10, 20, or 50-fold greater than the decrease in the number of non-cancerous cells. In yet another embodiment, the number of cancerous cells present after administration of a composition of the invention is at least 2, 5, 10, 20, or 50-fold lower than the number of cancerous cells present after administration of a vehicle control. Desirably, the methods of the present invention result in a decrease of 20, 40, 60, 80, or 100% in the size of a tumor as determined using standard methods. Desirably, at least 20, 40, 60, 80, 90, or 95% of the treated subjects have a complete remission in which all evidence of the cancer disappears. Desirably, the cancer does not reappear or reappears after at least 5, 10, 15, or 20 years. In another desirable embodiment, the length of time a patient survives after being diagnosed with cancer and treated with a composition of the invention is at least 20, 40, 60, 80, 100, 200, or even 500% greater than (i) the average amount of time an untreated patient survives or (ii) the average amount of time a patient treated with another therapy survives.

By "bacterial infection" is meant the invasion of a host animal by pathogenic bacteria. For example, the infection may include the excessive growth of bacteria that are normally present in or on the body of a animal or growth of bacteria that are not normally present in or on the animal. More generally, a bacterial infection can be any situation in which the presence of a bacterial population(s) is damaging to a host animal. Thus, a animal is "suffering" from a bacterial infection when an excessive amount of a bacterial population is present in or on the animal's body, or when the presence of a bacterial population(s) is damaging the cells or other tissue of the animal. In one embodiment, the number of a particular genus or species of bacteria is at least 2, 4, 6, or 8 times the number normally found in the animal. The bacterial infection may be due to gram positive and/or gram negative bacteria.

By "viral infection" is meant the invasion of a host animal by a virus. For example, the infection may include the excessive growth of viruses that are normally present in or on the body of a animal or growth of viruses that are not normally present in or on the animal. More generally, a viral infection can be any situation in which the presence of a viral population(s) is damaging to a host animal. Thus, a animal is "suffering" from a viral infection when an excessive amount of a viral population is present in or on the animal's body, or when the presence of a viral population(s) is damaging the cells or other tissue of the animal.

By "function of a cell" is meant any cell activity that can be measured or assessed. Examples of cell function include, but are not limited to, cell motility, apoptosis, cell growth, cell invasion, vascularization, cell cycle events, cell differentiation, cell dedifferentiation, neuronal cell regeneration, and the ability of a cell to support viral replication. The function of a cell may also be to affect the function, gene expression, or the polypeptide biological activity of another cell, for example, a neighboring cell, a cell that is contacted with the cell, or a cell that is contacted with media or other extracellular fluid that the cell is contained in. A detectable phenotype may include, for example, any outward physical manifestation, such as molecules, macromolecules, structures, metabolism, energy utilization, tissues, organs, reflexes, and behaviors, as well as anything that is part of the detectable structure, function, or behavior of a cell, tissue, or living organism.

By "apoptosis" is meant a cell death pathway wherein a dying cell displays a set of well-characterized biochemical hallmarks that include cytolemmal membrane blebbing, cell soma shrinkage, chromatin condensation, nuclear disintegration, and DNA laddering. There are many well-known assays for determining the apoptotic state of a cell, including, and not limited to: reduction of MTT tetrazolium dye, TUNEL staining, Annexin V staining, propidium iodide staining, DNA laddering, PARP cleavage, caspase activation, and assessment of cellular and nuclear morphology. Any of these or other known assays may be used in the methods of the invention to determine whether a cell is undergoing apoptosis.

By "polypeptide biological activity" is meant the ability of a target polypeptide to modulate cell function. The level of polypeptide biological activity may be directly measured using standard assays known in the art. For example, the relative level of polypeptide biological activity may be assessed by measuring the level of the mRNA that encodes the target polypeptide (*e.g.*, by reverse transcription-polymerase chain reaction (RT-PCR) amplification or Northern blot analysis); the level of target polypeptide (*e*.*g.*, by ELISA or Western blot analysis); the activity of a reporter gene under the transcriptional regulation of a target polypeptide transcriptional regulatory region (*e.g.*, by reporter gene assay, as described below); the specific interaction of a target polypeptide with another molecule, for example, a polypeptide that is activated by the target polypeptide or that inhibits the target polypeptide activity (*e.g.*, by the two-hybrid assay); or the phosphorylation or glycosylation state of the target polypeptide. A compound, such as a dsRNA or antisense nucleic acid that silences a repressor or a negative regulator, that increases the level of the target polypeptide, mRNA encoding the target polypeptide, or reporter gene activity within a cell, a cell extract, or other experimental sample is a compound that stimulates or increases the biological activity of a target polypeptide. A compound, such as a dsRNA or antisense nucleic acid, that decreases the level of the target polypeptide, mRNA encoding the target polypeptide, or reporter gene activity within a cell, a cell extract, or other experimental sample is a compound that decreases the biological activity of a target polypeptide.

By "modulates" is meant changing, either by a decrease or an increase. For gene silencing applications, a nucleic acid desirably decreases the function of a cell, the expression of a target nucleic acid in a cell, or the biological activity of a target polypeptide in a cell by least 20%, more desirably by at least 30%, 40%, 50%, 60% or 75%, and most desirably by at least 90%. For expression of a desired RNA or protein, a nucleic acid desirably increases the function of a cell, the expression of a target nucleic acid in a cell, or the biological activity of a target polypeptide in a cell by at least 1.5-fold to 2-fold, more desirably by at least 3-fold, and most desirably by at least 5-fold.

By "a decrease" is meant a lowering in the level of: a) protein (*e.g.*, as measured by ELISA or Western blot analysis); b) reporter gene activity (*e.g.*, as measured by reporter gene assay, for example, β-galactosidase, green fluorescent protein, or luciferase activity); c) mRNA (*e.g.*, as measured by RT-PCR or Northern blot analysis relative to an internal control, such as a "housekeeping" gene product, for example, β-actin or glyceraldehyde 3-pbosphate dehydrogenase (GAPDH)); or d) cell function, for example, as assayed by the number of apoptotic, mobile, growing, cell cycle arrested, invasive, differentiated, or dedifferentiated cells in a test sample. In all cases, the lowering is desirably by at least 20%, more desirably by at least 30%, 40%, 50%, 60%, 75%, and most desirably by at least 90%. As used herein, a decrease may be the direct or indirect result of PTGS, TGS, or another gene silencing event.

By "an increase" is meant a rise in the level of: (a) protein (*e.g.*, as measured by ELISA or Western blot analysis); (b) reporter gene activity (*e.g.*, as measured by reporter gene assay, for example, β-galactosidase, green fluorescent protein, or luciferase activity); (c) mRNA (*e.g.,* as measured by RT-PCR or Northern blot analysis relative to an internal control, such as a "houselceeping" gene product, for example, β-actin or glyceraldehyde 3-phosphate dehydrogenase (GAPDH)); or (d) cell function, for example, as assayed by the number of apoptotic, mobile, growing, cell cycle arrested, invasive, differentiated, or dedifferentiated cells in a test sample. Desirably, the increase is by at least 1.5-fold to 2-fold, more desirably by at least 3-fold, and most desirably by at least 5-fold. As used herein, an increase may be the indirect result of PTGS, TGS, or another gene silencing event. For example, the dsRNA or antisense nucleic acid may inhibit the expression of a protein, such as a suppressor protein, that would otherwise inhibit the expression of another nucleic acid.

By "alteration in the level of gene expression" is meant a change in transcription, translation, or mRNA or protein stability such that the overall amount of a product of the gene, *i.e.*, mRNA or polypeptide, is increased or decreased.

By "protein" or "polypeptide" or "polypeptide fragment" is meant any chain of more than two amino acids, regardless of post-translational modification (*e.g.*, glycosylation or phosphorylation), constituting all or part of a naturally-occurring polypeptide or peptide, or constituting a non-naturally occurring polypeptide or peptide.

By "under conditions that inhibit or prevent an interferon response or a dsRNA stress response" is meant conditions that prevent or inhibit one or more interferon responses or cellular RNA stress responses involving cell toxicity, cell death, an anti-proliferative response, or a decreased ability of a dsRNA to carry out a PTGS or TGS event. These responses include, but are not limited to, interferon induction (both Type 1 and Type II), induction of one or more interferon stimulated genes, PKR activation, 2'5'-OAS activation, and any downstream cellular and/or organismal sequelae that result from the activation/induction of one or more of these responses. By "organismal sequelae" is meant any effect(s) in a whole animal, organ, or more locally (*e.g.,* at a site of injection) caused by the stress response. Exemplary manifestations include elevated cytokine production, local inflammation, and necrosis. Desirably the conditions that inhibit these responses are such that not more than 95%, 90%, 80%, 75%, 60%, 40%, or 25%, and most desirably not more than 10% of the cells undergo cell toxicity, cell death, or a decreased ability to carry out a PTGS, TGS, or another gene silencing event, compared to a cell in which the same nucleic acid is introduced without using a composition of the invention (e.g., same cell type but not exposed to a targeting spermine and endosomolytic spermine or an oil-in-water emulsion of the invention).

Apoptosis, interferon induction, 2'5' OAS activation/induction, PKR induction/activation, anti-proliferative responses, and cytopathic effects are all indicators for the RNA stress response pathway. Exemplary assays that can be used to measure the induction of an RNA stress response as include a TUNEL assay to detect apoptotic cells, ELISA assays to detect the induction of alpha, beta and gamma interferon, ribosomal RNA fragmentation analysis to detect activation of 2'5'OAS, measurement of phosphorylated eIF2a as an indicator of PKR (protein kinase RNA inducible) activation, proliferation assays to detect changes in cellular proliferation, and microscopic analysis of cells to identify cellular cytopathic effects. In other embodiments, the level of an interferon response or a dsRNA stress response in a cell transformed with a nucleic acid using the methods of the present invention is less than 500%, 200%, 100%, 50%, 25%, or 10% greater than the corresponding level in a corresponding control, untransformed cell. Desirably, the double stranded RNA does not induce a global inhibition of cellular transcription or translation.

By "specifically hybridizes" is meant a dsRNA or antisense nucleic acid that hybridizes to a target nucleic acid but does not substantially hybridize to other nucleic acids in a sample (*e.g.*, a sample from a cell) that naturally includes the target nucleic acid, when assayed under denaturing conditions. In one embodiment, the amount of a target nucleic acid hybridized to, or associated with, the dsRNA, as measured using standard assays, is 2-fold, desirably 5-fold, more desirably 10-fold, and most desirably 50-fold greater than the amount of a control nucleic acid hybridized to, or associated with, the dsRNA.

By "substantial sequence identity" is meant sufficient sequence identity between a dsRNA or antisense nucleic acid and a target nucleic acid for the dsRNA or antisense nucleic acid to inhibit the expression of the nucleic acid. Desirably, the sequence of the dsRNA or antisense nucleic acid is at least 40, 50, 60, 70, 80, 90, 95, or 100% identical to the sequence of a region of the target nucleic acid.

By "substantial sequence complementarity" is meant sufficient sequence complementarity between a dsRNA or antisense nucleic acid and a target nucleic acid for the dsRNA or antisense nucleic acid to inhibit the expression of the nucleic acid. Desirably, the sequence of the dsRNA or antisense nucleic acid is at least 40, 50, 60, 70, 80, 90, 95, or 100% complementary to the sequence of a region of the target nucleic acid.

By "specifically inhibits the expression of a target nucleic acid" is meant inhibits the expression of a target nucleic acid more than the expression of other, non-target nucleic acids which include other nucleic acids in the cell or biological sample have a sequence that is less than 99, 95, 90, 80, or 70% identical or complementary to that of the target nucleic acid. Desirably, the inhibition of the expression of these non-target molecules is 2-fold, desirably 5-fold, more desirably 10-fold, and most desirably 50-fold less than the inhibition of the expression the target nucleic acid.

By "high stringency conditions" is meant hybridization in 2X SSC at 40°C with a DNA probe length of at least 40 nucleotides. For other definitions of high stringency conditions, see F. Ausubel et al., Current Protocols in Molecular Biology, pp. 6.3.1-6.3.6, John Wiley & Sons, New York, NY, 1994, hereby incorporated by reference.

By "expression element" is meant any feature or sequence of a DNA molecule that affects transcription or translation of a nucleic acid sequence. Examples of expression elements include promoters, enhancers, repressors, polyadenylation sites, and introns. Expression elements that can be assessed using this invention also include protein elements such as transcriptional or translational enzymes, for example, polymerases and transcription factors.

By "expression vector" is meant any double stranded DNA or double stranded RNA designed to transcribe an RNA, e.g., a construct that contains at least one promoter operably linked to a downstream gene or coding region of interest (*e.g.*, a cDNA or genomic DNA fragment that encodes a protein, or any RNA of interest, optionally, e.g., operatively linked to sequence lying outside a coding region, an antisense RNA coding region, a dsRNA coding region, or RNA sequences lying outside a coding region). Transfection or transformation of the expression vector into a recipient cell allows the cell to express RNA or protein encoded by the expression vector. An expression vector may be a genetically engineered plasmid, virus, or artificial chromosome derived from, for example, a bacteriophage, adenovirus, retrovirus, poxvirus, or herpesvirus.

By an "expression construct" is meant any double-stranded DNA or double-stranded RNA designed to transcribe an RNA, e.g., a construct that contains at least one promoter operably linked to a downstream gene or coding region of interest (*e.g.*, a cDNA or genomic DNA fragment that encodes a protein, or any RNA of interest). Transfection or transformation of the expression construct into a recipient cell allows the cell to express RNA or protein encoded by the expression construct. An expression construct may be a genetically engineered plasmid, virus, or artificial chromosome derived from, for example, a bacteriophage, adenovirus, retrovirus, poxvirus, or herpesvirus. An expression construct does not have to be replicable in a living cell, but may be made synthetically.

By "operably linked" is meant that a nucleic acid molecule and one or more regulatory sequences (e.g., a promoter) are connected in such a way as to permit transcription of the mRNA or permit expression and/or secretion of the product (i.e., a polypeptide) of the nucleic acid molecule when the appropriate molecules are bound to the regulatory sequences.

By a "promoter" is meant a nucleic acid sequence sufficient to direct transcription of a covalently linked nucleic acid molecule. Also included in this definition are those transcription control elements (*e.g.*, enhancers) that are sufficient to render promoter-dependent gene expression controllable in a cell type-specific, tissue-specific, or temporal-specific manner, or that are inducible by external signals or agents; such elements, which are well-known to skilled artisans, may be found in a 5' or 3' region of a gene or within an intron. Desirably a promoter is operably linked to a nucleic acid sequence, for example, a cDNA or a gene in such a way as to permit expression of the nucleic acid sequence.

By "reporter gene" is meant any gene that encodes a product whose expression is detectable and/or able to be quantitated by immunological, chemical, biochemical, or biological assays. A reporter gene product may, for example, have one of the following attributes, without restriction: fluorescence (*e.g.,* green fluorescent protein), enzymatic activity (*e*.*g.*, β-galactosidase, luciferase, chloramphenicol acetyltransferase), toxicity (*e.g.,* ricin A), or an ability to be specifically bound by an additional molecule (*e.g.,* an unlabeled antibody, followed by a labelled secondary antibody, or biotin, or a detectably labelled antibody). It is understood that any engineered variants of reporter genes that are readily available to one skilled in the art, are also included, without restriction, in the foregoing definition.

By "endosomolytic spermine" is meant a spermine that has been modified by the covalent attachment of a cholesterol or fatty acid and promotes the disruption of an endosomal vesicle. For example, the endosomolytic spermine may insert into the membrane of an endosomal vesicle and destabilize it. Desirably, the endosomolytic spermine increases the disruption of an endosomal vesicle by at least 20, 40, 60, 80, 100, 200, 500, 1000, or 5000% more than an unmodified spermine.

By "targeting spermine" is meant spermine that has been modified by the covalent attachment of a ligand for a cell surface molecule (e.g., a cell surface receptor, protein, or carbohydrate). The ligand may include, e.g., any molecule that can bind a cell surface molecule and trigger endocytosis. Desirably, the targeting spermine increases the introduction of the nucleic acid into a desired cell or tissue by at least 20, 40, 60, 80, 100, 200, 500, 1000, or 5000% more than an unmodified spermine. If desired, more than one ligand can be used in a composition of the invention (e.g., more than one ligand on the same targeting spermine, or more than one targeting spermine with a different ligand) to increase specificity for a desired cell or tissue.

By "positive to negative charge ratio" is meant the molar ratio of the number of positive charges in spermine to the number of negative charges in the nucleic acid of a composition of the invention. For this calculation, an unmodified spermine is considered to have 4 positive charges. A spermine molecule with one modification (e.g., the addition of a ligand for a cellular receptor, a fatty acid, a cholesterol, a linker, or a PEG) is considered to have 3.5 positive charges, regardless of the identity of the modification. For example, a spermine that has been modified by the addition of a positively charged, negatively charged, or uncharged group is considered to have 3.5 positive charges. Similarly, a spermine molecule with two modifications (e.g., the addition of two of the following groups: a ligand for a cellular receptor, a fatty acid, a cholesterol, a linker, or a PEG) is considered to have 3 positive charges, regardless of the identity of the modification. For example, a spermine that has been modified by the addition of two positively charged, negatively charged, uncharged groups, or any combination thereof is considered to have 3 positive charges.

### Advantages

The compositions and methods of the invention have a variety of advantages. For example, the compositions typically cause minimal, if any, toxicity in cells. In particular, the compositions have minimal effect on the rate of cell proliferation of cell cultures, indicating that the compositions cause minimal toxicity. The use of spermine-containing molecules as targeting molecules and endosomolytic molecules results in less toxicity than the use of other polyamines with more amine groups. Polyamines with a larger number of amine groups than spermine bind DNA with higher affinity and thus remain bound to the nucleic acid in the composition, possibly inhibiting the desired biological function of the nucleic acid after it is introduced into a cell or animal. Thus, spermine has a weaker, more desirable affinity for nucleic acids that facilitates the introduction of functional nucleic acids into cells and animals.

The use of one or more targeting spermines in the compositions of the invention improves the specificity of the compositions for desired cells or tissues within an animal. The use of endosomolytic spermines enhances the ability of the administered nucleic acids to be released from endosomal vesicles and exert their desired biological function. In contrast, in one traditional method for delivering nucleic acids to cells using a modified antibody; only 5% of cells expressed the encoded protein, although a high degree of specificity and efficiency of DNA, uptake were demonstrated in these studies (Mohr et al., Hepatology (1999) 29: 82-89).

The oil-in-water emulsions that contain nucleic acids in the oil phase protect the nucleic acids from harsh conditions, such as the acidic conditions of the stomach. Thus, oil-in-water emulsions that are delivered orally protect the nucleic acids within the oil phase from degradation.

### DETAILED DESCRIPTION

The current invention relates to compositions and methods for nucleic acid delivery, including methods and compositions for delivery of RNA, DNA, PNA, and hybrids thereof. In one aspect of the invention, the nucleic acid-delivery complex of the invention is designed to achieve delivery of double-stranded RNA (dsRNA) for either tissue specific silencing of a target gene or silencing of a target gene in the whole animal (RNAi).

One composition of the invention includes a nucleic acid, an endosomolytic spermine that includes a cholesterol or fatty acid, and a targeting spermine that includes a ligand for a cell surface molecule. The ratio of positive to negative charge of the composition is between 0.5 and 1.5, inclusive; the endosomolytic spermine constitutes at least 20% of the spermine-containing molecules in the composition; and the targeting spermine constitutes at least 10% of the spermine-containing molecules in the composition. The targeting spermine is designed to localize the composition to a particular cell or tissue of interest. The endosomolytic spermine disrupts the endosomal vesicle the encapsulates the composition during endocytosis, facilitating release of the nucleic acid from the endosomal vesicle and into the cytoplasm or nucleus of the cell.

Another composition of the invention includes a nucleic acid complexed with a cationic amphiphile in an oil-in-water emulsion (e.g., an emulsion with over 50% water) in which at least 10% of the complex is in the oil phase of the emulsion. Desirably, at least 25, 50,50, 70, 75, 80, 90, 95, 98, or 99% of the nucleic acid is in the oil phase of the emulsion. In various embodiments, the cationic amphiphile is a cationic lipid, modified or unmodified spermine (e.g., spermine modified with a hydrophobic group such as a fatty acid, including a C₃ to C₂₀ fatty acid, cholesterol, a fatty acid and a cholesterol, two fatty acids, or two cholesterols), bupivacaine, or benzalkonium chloride. The oil surrounding the nucleic acid in the composition protects the nucleic acid from harsh environmental conditions (e.g., the acidity of the stomach after oral delivery) after the composition is administered to an animal.
The improved nucleic acid delivery vehicles of the invention are designed to deliver nucleic acid compositions into cells. In addition to these naturally occurring materials, the nucleic acid compositions used in the present invention can also include synthetic compositions (e.g., nucleic acid analogs). These have been found to be particularly useful in antisense methodology, which is the complementary hybridization of relatively short oligonucleotides to single-stranded RNA or single-stranded DNA such that the normal, essential functions of the corresponding intracellular target nucleic acids are disrupted. The size, nature and specific sequence of the nucleic acid composition to be transferred to the target cell can be optimized, if desired, for the particular application for which it is intended, and such optimization is well within the skill of the artisan in this field.

In some applications, a nucleic acid expression construct capable of expressing a ribonucleic acid and/or a desired polypeptide is delivered. Such a nucleic acid composition has an appropriate open reading frame and promoter to express an RNA and/or protein, as well as any other regulatory sequences which may be appropriate to expression. Nucleic acid compositions to be delivered by means of the methods of the present invention can be designed and constructed so as to be appropriate for the particular application desired, all of which is well within the ordinary skill of the artisan in this field.

The nucleic acid molecules which are delivered to cells using the multifunctional molecular complex and methods of the present invention may serve as (1) genetic templates for proteins that function as prophylactic and/or therapeutic immunizing agents; (2) replacement copies of defective, missing or non-functioning genes; (3) genetic templates for therapeutic proteins; (4) genetic templates for antisense molecules and as antisense molecules per se; (5) genetic templates for ribozymes; (6) genetic templates for expression of dsRNA molecules; and/or (7) dsRNA molecules for RNAi applications.

In the case of nucleic acid molecules which encode proteins, the nucleic acid molecules desirably comprise the necessary regulatory sequences for transcription and translation in the target cells of the individual animal to which they are delivered. Nucleic acid molecules which serve as templates for antisense RNA, dsRNA molecules, or ribozymes are desirably linked to regulatory elements necessary for production of sufficient copies of the encoded antisense, and ribozyme molecules. These nucleic acids are desirably provided in the form of plasmids.

The nucleic acid compositions used in the present invention may be either single-stranded or double-stranded, may be linear or circular (e.g., a plasmid) and may be oligo- or polynucleotides. The nucleic acids may comprise as few as 5-10 bases or base pairs, or may include as many as 45 thousand bases or base pairs (45 kb). Since the transfer moiety is employed on a proportional basis when added to the nucleic acid composition, practical considerations of physical transport and the ability to isolate intact molecules from bacteria may largely gavern the upper limit on the size of nucleic acid compositions which can be utilized.

### Summary of Traditional Methods for Condensation of Nucleic Acids and Entry into the Cell

Efficient *in vivo* transfection remains a challenge for nucleic acid based therapies. "Naked" DNA can be administered and used to transfect cells *in vivo,* albeit poorly due to problems which are likely the result of DNA being negatively charged and binding proteins and other molecules having cationic side chains. Numerous *in vitro* studies have shown that DNA enters cells following neutralization of the charge. Multivalent cations and cations that have multiple coordination spheres, e.g., polyamines and cobalt hexa- and penta-amines interact with the DNA molecule in more ways than simply forming a salt bridge with the phosphoryl group. Such cationic compounds not only charge neutralize the DNA but also hydrogen bond with the bases, promoting condensation of DNA. Thus, condensation of DNA is thought to be one of the prerequisites for efficient and predictable transfection.

Transfection into a cell requires the use of agents and methods that facilitate entry of the charged nucleic acid. Most transfection methods are passive and rely on the use of complexing agents such as cationic lipids, DEAE dextran, or Ca⁺⁺. Following nearly a decade of research in transfection, it is clear that these methods of nucleic acid transfer do not transfect cells *in vivo* at a level sufficient to be biologically relevant. Thus, for nucleic acid based therapeutics to be successful, improved passive transfection technologies and active transfection technologies that invoke cellular participation must be developed.

### Summary of Traditional Methods Using Cationic Lipids and Water Soluble Cationic Amphiphiles

Cationic amphiphiles are ion-pair molecules that comprise both lipophilic and hydrophilic domains. The relative amounts af hydrophobicity and the ionic component of the molecule determines its desirable solubility properties. In an aqueous environment, a cationic amphiphile having a single lipid chain and a cationic head group tends to form a micelle structure, with the lipid chains oriented into a hydrophobic core. Micelles do not have an internal volume and therefore are unable to trap drug molecules to significant levels. Moreover, upon entry into circulation *in vivo* micelles are preferentially taken up by macrophage-like cells, such as Kupfer cells in the liver. Rapid degradation of DNA is anticipated in the hostile intracellular environment of such cells; therefore, micelle delivery of nucleic acids is not desired.

Certain cationic amphiphiles comprise two lipid chains and a cationic headgroup. A double chain lipid with appropriate hydrocarbon chain length can form bilayered vesicles. Even if the solvent contained within the vesicle were identical to the external solvent, the vesicle is expected to show large amounts of asymmetry (heterogeneity), possibly due to differences in the packing order of the lipid between the two layers. A much more complex structure is formed when DNA or a co-lipid is added. These multi-lipid interactions bring about local rigidity in an otherwise fluid environment of a mono-lipid vesicle, resulting in the reassortment of the layers and further increasing the asymmetry of the complex. Heterogeneity in bilayers is predicted to result in heterogeneous interactions with the molecule intended to be delivered, resulting in heterogeneity of complexes, and affecting efficiency and reproducibility of transfection and the ultimate desired effect.

While lateral fluidity exists to a certain extent in a bilayer, there are no reports of lipid exchange between the layers, possibly due to structural stability of the two layers in a bilayer. Similar thermodynamic obstacles exist in getting two liposomes to fuse, and even more so in getting them to fuse with cellular membranes. Numerous reports have shown that liposomes do not fuse with cellular membranes; rather the uptake is through a passive phagocytic process. Translocation of phosphatidyl lipids from added liposomes to cells has been shown to be protein mediated. However, it is predicted that a controlled translocation of phosphatidyl lipids could change the thermodynamic equilibrium to favor fusion. Entry of DNA into a cell appears to be facilitated through increased residence time near an actively phagocytic cell or through complexes that contain functional groups that actively interact with membrane components to reorder the local membrane structure, such as in receptor mediated targeting.

It is therefore not surprising that cationic lipid-DNA complexes have in general performed less well than naked DNA. It has been shown that cationic lipid complexes of DNA aggregate in culture and that an aggregation level of three microns induced the phagocytic (or pinocytic) response of the cell, resulting in transfection. However, larger aggregates were inhibitory, suggesting that there is a limited window of opportunity for transfection, based on the size of the complex. The non-transfectability of cationic lipid-DNA complexes *in vivo* is often rationalized as involving possible cationic lipid interactions with other molecules such as proteins in serum, thus altering the idealized transfecting particle created *in vitro.*

Cationic lipid complexes that have been designed to fuse with cellular membranes, and deliver the associated DNA intra-cellularly are desired. There is no evidence that lipid bilayers fuse with cellular membranes as a biochemical mechanism in an equilibrium state. Bilayer structures could play a role, but only after reordering (lipid phase transition) of both the incoming bilayer and the cellular bilayer, possibly through other interactions, resulting in translational motion and possibly phase separation followed by solidification of the membrane. However, cell membranes are heterogeneous in a particular cell and across cell types. They contain lipids, carbohydrates, and proteins. Carbohydrates are present as glycoproteins and glycolipids, and their content varies from 1.5-10% in different cell types, while the protein to lipid ratio varies between 0.2 in myelin to 1.5% in liver cells. This heterogeneity suggests that a singular solution for all these complexities may be difficult to define. In conclusion, liposomes have not been shown to be actively fusogenic.

### Summary of Traditional Methods Using Vesicular Cationic Amphiphile Complexes for RNAi Applications

As compared to cationic lipids, simple structures derived from water-soluble amphiphiles have a higher probability of forming transfecting structures that are uniform and homogenous, which is expected to result in increased efficiency, predictability, and potency. We selected cationic amphiphiles that are membrane active, such as the local anesthetics, e.g., bupivacaine (see U.S. 6,383,512, "Vesicular complexes and methods of making and using the same," which describes compositions comprising lamellar vesicles that comprise a local anesthetic, e.g., bupivacaine, and a nucleic acid molecule). In particular, this reference describes compositions and methods for delivery of nucleic acid molecules that are antisense, ribozyme, or triplex forming nucleic acid molecules, as well as nucleic acid molecules that encode proteins (e.g., non-immunogenic therapeutically effective proteins, and immunogenic proteins designed to elicit a desired immune response in a host). Experiments were carried out to determine whether such vesicular complexes could be used to deliver dsRNA expression constructs capable of eliciting RNAi. Local anesthetics, unlike cationic lipids, actively interact with membranes and have been developed to possess properties such as decreased interactions with serum proteins and increased membrane permeability. Additionally, these are currently used in humans, and their toxicity / dose profiles are well characterized. In contrast, the associated toxicity of lipids that transfect effciently *in vitro* may preclude their use *in vivo.* However, water-soluble cationic amphiphiles such as local anesthetics (i) show little or no toxicity at concentrations required to achieve efficient transfection *in vivo,* (ii) transfect cells *in vivo* to a greater extent than naked DNA, and (iii) can be ordered to form homogenous complexes of uniform size and charge distribution that increase their potency. Different types of complexes can be prepared through variations in the method and controlled rate of assembly. While it has been observed that local anesthetic complexed DNA does not transfect cells efficiently *in vitro* (at <1% of a lipofectamine transfection efficiency), such complexes transfect cells *in vivo* resulting in expression levels that are over 3 fold better than DNA without transfecting agents. Although, naked DNA transfects cells *in vivo,* it has been shown to be ineffective in *in vitro* transfections.

### Nucleic Acid Delivery Methods of the Invention

The methods described herein may be used (1) to develop DNA complexes of polyamine based water-soluble amphiphiles, cholesteryl polyamines, (2) to develop transfection methods utilizing cholesteryl polyamines (*in vitro* and *in vivo*) utilizing complexes with plasmid DNA, (3) to deliver proteins along with DNA by entrapping proteins within these complexes, and (4) to develop DNA complexes partitioned into oils for oral delivery and for delivery into tissues, such as muscle.

Bis and monocholesteryl derivatives of spermine and spermidine have been synthesized. Cholesterol was attached to the spermine molecules at the 3-OH position, linked through a 6-10 carbon atom spacer (carbamoyl or amide linkage) to the secondary nitrogen of spermines. The compound was shown to be >98% pure by mass spectrometry and NMR. Elemental analyses have also been performed. These compounds have been shown to complex with DNA by gel shift analysis, electron microscopy and photon correlation spectroscopy. The physical nature of the complexes may be varied by varying concentrations of the reactants and conditions of the complexation process, presumably through perturbation of the critical rates of the reaction pathway. The observations made in our discovery of the local anesthetic complexation of DNA may be extended to complexes formed with cholesteryl polyamines. The condensation process may be monitored by UV, IR, CD, and/or photon correlation spectroscopies. Electron microscopy and sedimenation analyses may be performed to determine the homogeneity of the complex. Interactions with serum components may also be assessed. Stability of these complexes, the DNA, the transfecting reagents, and the aggregation state may be assessed for their physical properties before advancing them to biological studies.

Physically distinct complexes in size, charge density, and hydrophobicity may be analyzed for transfectability of cells. Using a reporter gene that encodes, e.g., the secreted heat stable human placental alkaline phosphatase, serum levels may be measured to assess transfection efficiency. *In vitro* studies may utilize human rhabdomyosarcoma cells in culture and mice for *in vivo* studies. Preliminary data show that these complexes transfect cells *in vitro* at similar efficiencies as commercially available transfecting agents (cationic lipids) and *in vivo* at efficiencies equal to that of the local anesthetic complexes. Further optimization of the complexation process can be used, if desired, to further increase the transfection efficiencies in *in vitro* and *in vivo.*

In our DNA complexation studies with the local anesthetic bupivacaine, it was shown that these DNA complexes are homogenous and that unimolecular complexes can be prepared by controlling the rate of interaction. It was also demonstrated that these complexes contain internal water and can be made to contain a variety of water-soluble molecules (e.g., large and small molecules including enzymes). Plasmid distribution studies have indicated that only a fraction of the internalized DNA molecules enter the nuclear compartment and are transcribed. Therefore, harnessing the transcription potential of the majority of plasmid molecules located in the cytoplasm is desired to achieve higher levels of expression. However, the lack of RNA polymerase in the cytoplasm precludes utilization of these internalized plasmid molecules. Co-delivery of cytoplasmically active RNA polymerases entrapped in complexes is predicted to initiate the expression cycle. Co-administration of cytokines and chemokines to augment an immune response to a DNA vaccine is also a desired application.

Extending our observations that complexes made with bupivacaine allow partitioning of DNA into hydrophobic solvents, formulations may be developed that are lipid based and that are used for delivery to mucosal compartments to induce mucosal immune responses with DNA based vaccines, and for delivery into muscle where a lipid based formulation may be expected to allow slow release of the contained DNA, thereby altering the pharmacokinetics of the injected DNA and its bioavailability. We have shown previously that within hours of inoculation, uncomplexed DNA levels drop a million fold and drop a further 10,000 fold in a week.

### Microemulsions Based Delivery of DNA Molecules

Improved methods for intracellular delivery of genes are desired. In particular, vehicles are needed to carry and deliver nucleic acids, including plasmid DNA molecules, to cells *in vivo* for a variety of therapeutic applications, including RNAi, antisense, gene therapy, and genetic vaccine applications. Current progress in nucleic acid therapeutics is limited by the ability to deliver DNA and RNA into cells. Microemulsions are predicted to enhance intracellular delivery. The commercial prospects of nucleic acid therapeutics are linked directly to the success of such efficient delivery systems.

One approach to deliver genes *in vivo,* particularly by oral route, is stable microemulsion based delivery. Microemulsion based delivery has a high potential to deliver DNA to the mucosal compartment and through the portal circulation into the liver, or simply absorbed through the intestinal walls into general circulation. Delivery to the mucosal inductive sites may be the only approach to induce a mucosal response. A mucosal immune response is desired to prevent pathogen intrusion, particularly pathogens responsible for STDs. Furthermore, due to absorption through mucosal surfaces, systemic delivery of DNA can also be effected. Systemic transfection that follows absorption can facilitate gene therapy and RNAi applications.

Microemulsions are submicron particles (5-100 nm) formed by a self-assembly process which is solvent driven. Thermodynamically stable microemulsion is a nanotechnological method to develop structures that readily permeate cells to deliver drug molecules that are entrapped in them. Architecture of these particles is key to their function, while solvent interactions determine the architecture of these micro-carriers. Nucleic acid drug molecules (e.g., DNA or oligonucleotides) are predicted to partition into the desirable and accessible solvent phases. In a typical water-in-oil emulsion, the hydrophilic nucleic acid molecules are predicted to be in the water phase of the particle. Unlike conventional emulsions, microemulsions are thermodynamically stable drug carriers. However, the solvent interactions are readily reversed to deliver their contents within the cells. Inclusion of membrane active agents in either phase is predicted to give unique delivery characteristics to these mico-carriers.

Methods described herein involve the development of a generalized transfection approach for rapid delivery of nucleic acids such as DNA into cells, through the use of permeation enhancers in stable microemulsions (MEs). The compositions are predicted to allow for slow release of DNA from a depot (injection site), oral administration of plasmid DNA based vaccines, gene therapy, and RNAi applications. A strong commercial interest exists for the delivery of DNA for gene therapy and genetic vaccine applications, particularly those that can be administered orally. These emulsions are particularly favored for oral delivery because the DNA is protected in the acid environment of the stomach, since the DNA is contained in the internal hydrophilic microenvironment of the emulsion, while the oil is exposed to the macro acid environment. The damage to DNA (through acidification) is predicted to be rate-limited by the rate at which the gastric fluid phase separates into emulsions themselves and fuses with DNA containing emulsions. MEs are also anticipated to favor unique biodistribution and pharmacokinetic parameters to DNA vaccines, as opposed to those observed with uncomplexed DNA or delivered by other means. Membrane activities of the components in a microemulsion are also predicted to allow rapid absorption through membranes.

Microemulsions have been defined as clear, transparent and stable isotropic mixtures of oil, water, surfactants, and cosurfactants. Their major difference from conventional emulsions is the small size of the dispersed phase droplets (5-200 nm), which is responsible for their optical clarity. Microemulsions have been used as oil-in-water (o/w) carriers for poorly soluble drugs as well as water-in-oil (w/o) carriers for water-soluble drugs. Microemulsions are thermodynamically stable and provide improved drug solubilization and protection against enzymatic hydrolysis of unstable water soluble drugs in a water in oil emulsion. At the cellular level, these lipid based formulations (MEs) are designed to interact with the mucosal membranes, changing membrane fluidity and thus permeability. Microemulsions can diffuse through tight junctions intercellularly or are taken up by endocytosis. Because of their size, pinocytosis and carrier mediated endocytosis can occur via Peyer's patches, upon oral administration.

Most absorption enhancers act by creating disorder in the phospholipids in the membrane. However, considerations of safety and mucosal damage may preclude their use in some instances. Some absorption enhancers that are apparently safe and do not induce major tissue damage have been investigated by Hastewwell *et* a/. In the search for safe and effective absorption enhancers, the trend is towards the use of materials based on natural substances such as glycerides. Glycerides have been investigated as absorption enhancers of many different drugs. Medium chain (mono-, di-, and tri-) glycerides (C8-C10) especially the mono- and diglycerides are considered good permeation enhancers that are also suitable for microemulsion formulation. Constantinides *et al.* have reported enhanced bioavailability of an RGD peptide compared to that from an aqueous formulation (from 0.5% to 27%) from a microemulsion formulation incorporating medium chain glycerides, which do not induce gross changes in the G.I. mucosa. *In vitro* studies demonstrate that medium chain glycerides markedly affect the permeability of paracellular markers. Smith and Ellens have studied the observed *in vitro* the effect of medium chain glycerides on rabbit intestinal epithelium, and suggested that absorption enhancement resulted from increased permeability of the intestinal cells confined to the villus or surface epithelium. The medium chain glycerides were found to affect the electrical properties and the permeability of the rabbit intestinal epithelium *ex vivo.* These effects are possibly mediated by MEs by decreasing ion-transport across the membrane. At high doses, MEs also caused an increase in solute transport by sloughing off cells from the surface epithelium. The distal colon was found to be more sensitive to the effect of medium chain glycerides than the ileum. Their results were similar to those reported earlier and the authors suggest further investigation to confirm the hypothesis. A more direct assessment of intestinal absorption of drugs using the mesenteric vein delivery in rat model has been reported, which checks the disappearance of a peptide from the lumen and appearance in mesenteric plasma. Results indicate a considerably higher mesenteric plasma delivery of the peptide from a microemulsion formulation compared to that from an aqueous citrate formulation. A Caeo2 cell system has been utilized to mimic intestinal tract conditions and to evaluate safety and the effectiveness of drug delivery, but did not show any gross morphological changes compared to that with isotonic pH 6 buffer.

Two types of emulsions, oil in water and water in oil, may be developed to incorporate plasmid DNA. This drug delivery system has several of the above mentioned advantages that result in efficient gene delivery to cells. Because of its inherent characteristics, DNA MEs (microemulsions) can be used to target M-cells (antigen presenting cells) in Peyers' patches for efficient oral delivery, and thus to generate a strong mucosal response.

### Types of Microemulsions

Water in oil stable microemulsions may be developed that transfect cells *in vitro.* Oral and intramuscular delivery using water in oil MEs may be performed in mice. Oil in water stable microemulsions, with DNA in the oil phase, may be generated and tested in mice.

### 1. Water in Oil MEs

The water in oil microemulsion may be developed by titrating a mixture of the oil, surfactant and cosurfactant (in a fixed ratio) with the aqueous phase and determining the points at which turbidity occurred. The water in oil nature of the microemulsions so prepared is confirmed by observing properties such as non dispersability in water, solubility of a water soluble dye (calcein), low conductivity values, and zeta potential. Phase diagrams with several components are constructed to identify ME fields, and the several desirable formulations for oral/parenteral delivery are selected.

Composition of one such desirable microemulsion for oral delivery may contain Lauroglycol ^{™} (propylene esters), Labrasol^{™} (saturated polyglycolyzed C₈-C₁₀ glycerides), ethanol, and plasmid DNA. These are analyzed for DNA uptake and expression in RD and/or Caco2 cells. Optimized MEs may be selected for oral admininistration in animal studies (e.g., mouse studies). In order to increase loading of DNA into MEs, and to facilitate condensation of the plasmid DNA molecules, cationic detergents may be tested. Benzalkonium chloride, a detergent, was shown to bind and condense DNA. Similarly, for parenteral use, alternate MEs may be generated. These MEs differ from the previously described MEs in that the components, particularly the permeation enhancers do not cause tissue injury. Soybean oil, cremaphor, and benzalkonium chloride may be used to develop these MEs as vehicles for gene delivery.

Using stained or gold particle attached DNA, the entrapment of DNA is assessed. Furthermore, photon correlation spectroscopic studies and phase transition analyses may be carried out. Preliminary data show that a 20 nm emulsion particle containing DNA can be prepared using short chain glycerides with a co-solvent and a detergent. The physical and functional characteristics of the MEs, may be assessed. Besides the biophysical attributes of the MEs the functionality of the DNA may also be assessed.

### 2. Oil in Water MEs

A recent discovery has allowed us to make novel emulsions. Cationic amphiphile complexed plasmid DNA was shown to partition into non-polar solvents. Extractability of the DNA molecule into hydrophobic solvents allows development of technologies that result in compartmentalization of DNA into the oil phase in an "oil in water" (o/w) emulsion. Such emulsions are predicted to give added protection to the DNA if administered orally, particularly from the stomach acids. These particles are also predicted to impart stability to DNA by decreasing accessibility to enzymes, and may also release the contained DNA more slowly over a longer period.

### Cell Type Specific Transfection Methods

Novel approaches for intracellular delivery of genes are desirable. In particular, vehicles that carry plasmid DNA molecules compacted through condensation and delivered to relevant cells *in vivo* are needed for gene therapy, gene silencing, and genetic vaccine applications.

Optimal delivery of genes can be achieved through the use of viral vectors. However, viral vectors induce immune responses to the vehicle itself, and undesired host responses as well. Non-viral gene delivery is therefore a desirable approach to deliver genes. Non-viral vectors are also predicted to be more stable than viral vectors. We have focused on several approaches to deliver genes *in vivo,* one of which is the active transfection approach that is targeted to a cell type. The particles are created through a self assembly process, in which a multifunctional structure is formed. This novel approach to delivering DNA is described herein. These micro particles transfect the desired cell type to a greater extent than passive methods of transfection. Functionally, these multifunctional particles have the characteristics of a virus in that these particles are preferentially internalized by the targeted cell type by mimicking a ligand to a cell membrane protein, thereby achieving transfection by utilizing cellular processes.

The delivery system described here results in submicron structures (20-100 nm particles) that are created by a self-assembly process, entirely driven through physical interactions between a nucleic acid molecule and the two different polyamine molecules that complex with it to form the particle, i.e., a receptor-specific polyamine liganding molecule and an endosomolytic polyamine molecule that perturbs the endosomal membrane. The architecture of these particles is the key to their functionality. In the assembly process, the monomeric units utilized to build the nanoparticle and the rate of self-self-self-assembly dictate the architecture, and thus the function of these particles. In these instances the monomeric units have been created based on their abilities to interact with DNA and thus to increase DNA loading into these micro carriers, thereby forming structures that include structural participation of the DNA molecule itself. All of the individual interactions between the monomeric units of the delivery vehicle and the nucleic acid are extremely weak and are readily reversed.

### DNA Complexation by Polyamines

Polyamines and cobalt hexa- and penta-amines not only charge neutralize the DNA but also hydrogen bond with bases. Condensed DNA is one of the prerequisites for transfection, and DNA condensation in cells is mediated through polyamine interactions. DNA complexes of cationic polypeptides also condense DNA efficiently and transfect cells *in vitro* and *in vivo.* However, certain polypeptide complexes of DNA have been shown to be immunogenic in animals. Therefore, polyamines offer certain advantages for condensation of DNA for transfection purposes; e.g., they can be chemically modified readily to contain biophysically and biochemically reactive appendages, they are the physiological counter-ions in a cell, they are generally non-toxic, and they are readily metabolized by oxidases in the cell. In addition, modifications of the secondary amines through a spacer arm do not significantly affect the electrostatic binding affinity of polyamines for DNA. Gel retardation of plasmid DNA is effected when complexed with derivatized spermines, such as mannosyl spermine or cholesteryl spermine.

### Targeted and Active Transfections

Targeted and active delivery of DNA and other nucleic acids appears to be a desirable transfection method, effecting higher transfection efficiencies. An active transfection is likely to ensure both the preservation of DNA during the transfection and cellular participation. To this end, targeting reagents that are based on polyamine-derived small molecules have been developed to facilitate that introduction of nucleic acids into cells and animals and the release of the nucleic acids from endosomal vesicles.

### Multifunctional polyamine based targeted transfecting systems

A multifunctional targeting system is an attempt to simulate the functional entities of particles, such as viruses that readily enter into the cytoplasm of cells. In the absence of knowledge of the structural entities and particle architecture that give rise to specific functions, we designed conditions to generate co-complexes of DNA using multiple polyamine molecules. Formulations and conditions that resulted in multifunctional co-complexes were identified through repeated screening *in vivo.* We have demonstrated that singular liganded spermine, complexes that target to the mannose receptor and to the asialoglycoprotein receptor result in rapid internalization of DNA into endosomal vesicles. However, these complexes were further designed to contain multiple functionalities, not only to enable receptor binding and internalization, but also endosomal escape. These multifunctional co-complexes were efficient and specific in transfecting the target cell types both *in vivo* and *in vitro.*

Our targeting system is based on a polyamine backbone, more particularly, a spermine backbone. We have developed two types of spermine derivatives, one derivative that has a targeting ligand bound to a nitrogen atom of the polyamine, and the other derivative that has an endosome membrane disruption promoting component (e.g., cholesterol or a fatty acid) bound to a nitrogen atom of the polyamine. The targeting ligand and the endosome membrane disruption promoting component may be attached to its respective polyamine through a suitable linker group, e.g., through a PEG spacer arm or e.g., through an alkyl, carboxamide, carbamate, thiocarbamate, or carbamoyl bridging group. The endosomolytic molecule and the targeting molecule are each attached though a selected linker to a secondary nitrogen of a spermine molecule. It was rationalized that mixing the two different spermine derivatives in different ratios may form differently architectured complexes with DNA. Some of these architectures may have the desired bio-active properties, e.g., efficient and selective nucleic acid uptake and endosomal escape. The nucleic acid complexes are predicted to be multifunctional (exhibiting both the targeting and endosomolytic properties) because both of the spermine derivatives may interact with the DNA molecule similarly and simultaneously.

Any of the ligands, linking groups, or synthetic methods described in U.S. 5,837,533; U.S. 6,379,965; or U.S. 6,127,170, which are hereby incorporated by reference, can be used to create compositions of the invention. Exemplary linking groups include alkyl, carboxamide, carbamate, thiocarbamate, or carbamoyl bridging groups. In some embodiments, the composition includes a fusogenic peptide comprising spike glycoproteins of enveloped animal viruses or cholic acid or cholesteryl or derivatives. Desirable targeting spermines include one or more receptor specific binding components which are ligands for natural receptors of the target cell, attached through, e.g., an alkyl, carboxamide, carbamate, thiocarbamate, or carbamoyl bridging group.

### Mannosyl Spermine targeted to the Mannose receptor of APCs

Monomannosyl and bismannosyl spermines were used to condense DNA, along with cholesteryl spermine molecules. Based on our previous studies, inclusions of cholesterol moieties (or other membrane active agents) were deemed necessary to enable escape from the internalized endosome prior to its maturation to lysosomes. Cholesterol induces membrane rigidity. Loss of fluidity of vesicular membranes by cholesterol has been shown to induce eversion. It was therefore predicted that cholesterol will interfere with endosome maturation and release DNA into the cytoplasm. A small, but proportionate amount of cytoplasmic DNA translocates into the nucleus, thereby facilitating nuclear transcription. The use of a multifunctional transfecting particle prepared by co-complexing spermine based endosome disruptors (e.g., cholesterol, low pH Detergents - LPHD) with the targeting spermine derivative (bismannosyl for APCs and trilactosylamine for hepatocytes) and plasmid DNA may be performed.

Only specific co-complexes of bismannosyl spermine and cholesteryl spermine with appropriate surface / chemical architecture transfect cells derived from the peritoneum *in vivo* and *in vitro.* All of the transfected cells contained mannose receptor, and all the cells that contained mannose receptor were transfected demonstrated specificity and a high efficiency. Other co-complexes may include other functionalities such as spermines modified to contain endosomal escape agents (LPHD).

### 2. Trilactosyl spermine to the ASG Receptor of hepatocytes

Trilactosyl oligonucleotides and trilactosylated monoclonal antibodies are rapidly (in minutes, in a singular pass) taken up by hepatocytes. Spermine derivatives that contain lactosyl groups have been developed to deliver DNA into hepatocytes. Preliminary experiments indicate a non-saturating uptake of labeled lactosylated-oligonucleotides to HepG2 cells. Co-complexes similar to the mannosyl-cholesteryl spermine co-complexes may be prepared and used to transfect hepatocytes *in vivo.* Information from co-complexes of either targeting system may be used in active and targeted transfections.

### The Design of the Multifunctional Transfecting Particle

Receptor mediated delivery to target cells is well known in the art, e.g., in U.S.5,837,533, the teaching of which is incorporated herein by reference. These approaches take advantage of natural receptor-mediated endocytosis pathways that exist in cells. Several cellular receptors have been identified as desirable agents for specific targeting of drugs, and especially macromolecules and molecular conjugates serving as carriers of genetic material of the type with which the present invention is concerned. These cellular receptors allow for specific targeting by virtue of being localized to a particular tissue, by having an enhanced avidity for a particular tissue, or by having an enhanced activity in a particular tissue (e.g., Bodmer and R. T. Dean, Meth. Enzymol., 112,298-306 (1985)). This affords the advantages of lower doses or significantly fewer undersirable side effects.

One of the better known examples of a cell and tissue selective receptor is the asialoglycoprotein receptor present in hepatocytes. The asialoglycoprotein receptor is an extracellular receptor with a high affinity for galactose, especially tri-antennary oligosaccharides, i.e., those with three somewhat extended chains or spacer arms having terminal galactose residues (e.g., H. F. Lodish, TIBS, 16, 374-77 (1991)). This high affinity is localized to hepatocytes and is not present in Kupffer cells, allowing for a high degree of selectivity in delivery to the liver.

It has also been proposed in the art of receptor-mediated gene transfer that in order for the process to be efficient *in vivo,* the assembly of the DNA complex should result in condensation of the DNA to a size suitable for uptake via an endocytic pathway (see, e.g., Perales et al., Proc. Nat. Acad. Sci. USA, 91, 4086-4090 (1994)).

An alternative method of providing cell-selective binding is to attach an entity with an ability to bind to the cell type of interest; commonly used in this respect are antibodies which can bind to specific proteins present in the cellular membranes or outer regions of the target cells. Alternative receptors have also been recognized as useful in facilitating the transport of macromolecules, such as biotin and folate receptors [see Low, Horn, and Heinstein, WO 90/12095, published 18 Oct. 1990; Low, Horn, and Heinstein, WO 90/12096, published 18 Oct. 1990; Low, Horn, and Heinstein, U.S. Pat. No. 5,108,921, Apr. 28, 1992; Leamon and Low, Proc. Nat. Acad. Sci. USA, 88, 5572-5576 (1991)] transferrin receptors, insulin receptors, and mannose receptors (see further below). The enumerated receptors are merely representative, and other receptors are well-known by skilled artisans.

### Targeting Antigen Presenting Cells (APCs) Ex Vivo and In Vivo Studies

In one aspect of the invention, the goal is to develop a multifunctional particle that targets nucleic acid delivery to antigen presenting cells, such as macrophages. Macrophage cell membranes contain the mannose receptor. Therefore the liganding spermine was designed to contain mannose as the targeting ligand. For the ease of synthesis, bis-mannose-spermines were prepared initially. We chose cholesterol as the endosome disrupting moiety. As stated above, biological membranes are at an equilibrium state. Although added lipids have no membrane disrupting potential in stationary membranes, endosomal membranes undergo changes as the endosome matures into a lysosome. The potential to disrupt membranes exists when membranes reorder, such as in a maturing endosome. Cholesterol is usually associated with membranes. A planar molecule such as cholesterol provides local rigidity to the otherwise fluid environment of a membrane. However, an overabundance of cholesterol in membranes is disruptive, as it interferes with lateral fluidity. Therefore, we chose cholesteryl spermine as the endosomolytic polyamine agent. Cholesterol was attached to the spermine molecules at the3- 3-OH position, linked through a 6-10 carbon atom spacer (carbamoyl or amide linkage) to the secondary nitrogen of spermine. The compound was shown to be >98% pure by mass spectrometry and NMR. Elemental analyses have also been performed.

Peritoneal fluid contains large amounts of antigen presenting cells, macrophages and dendritic cells. Peritoneal fluid was isolated from mice. Plasmid DNA designed to express the green fluorescent protein (GFP) in mammalian cells was complexed with a mixture of the two spermines, mannosyl-mannosyl-spermine and cholesteryl spermine, at different ratios. The complexes were applied to peritoneal cells in culture. The fluorescence intensity of the cells were recorded. The complexes that were prepared for the *ex vivo* study were injected into the peritoneal cavity of mice. Four and five days following the injection, the cells were collected from the peritoneal cavity and spun down on slides and their ability to fluoresce was determined. The fluorescence intensities were recorded visually. Transfection is afforded only by particles comprised of certain ratios of cholesteryl spermine and mannosyl spermine.

### Identification of the targeted cell type

Labeled antibodies to defined cell surface markers were used to identify the cell type. Most of the cells transfected (>95%) were macrophages. Some dendritic cells were also transfected. However, only about 80% of the macrophages were transfected, presumably because some lacked the mannosyl receptor, or these cells were not accessible to the complex. Using labeled DNA it has been shown that in all of the tested ratios, particles that contained the mannose liganded spermine were internalized. However, expression from the internalized plasmid was observed only when cholesteryl spermine was included in the architecture of the DNA polyamine particle, and only when sufficient amounts were represented in the particle. This result suggests that mannose spermine causes receptor-specific cellular uptake of DNA to which it is complexed, but that the DNA complex must also contain sufficient amounts of cholesteryl spermine in order for the DNA to be released from the endosome into the cell where expression can occur.

### Exemplary Conditions and Rangers

In applications where DNA expression constructs are to be utilized, plasmids are a desirable embodiment, as is supercoiled DNA. The DNA can be linear, open circular, or sheared. A desirable positive to negative charge ratio is between 0.5 and 1.5, inclusive. Desirably, the positive to negative charge ration is between 0.8 and 01.2, inclusive. The optimal ratio of targeting spermine (desirably a mannosyl, lactosyl, folate, or biotinylated spermine) to endosomolytic spermine (e.g., LPHD or cholesteryl spermine) is 0.35 : 0.65 (molar ratio). An acceptable range of targeting spermine to endosomolytic spermine is 0.10 : 0.90 to 0.50 : 0.50. Mixtures are prepared in a buffered solution containing, e.g., 30 mM citrate (pH 6.9) (buffers may be e.g., citrate, succinate or phosphate and can range from 5 to 50 mM and from pH 6.0 to 8.2). EDTA may be included at 0.1 mM (or up to 5 mM), NaCl may be included from 1 mM to 200 mM. Desirably, the final DNA concentration is 1 mg / mL; the range can be 10 ug / mL to 10 mg /mL. Injection volumes are desirably at 100 uL; the range can be 10 uL to 1.0 mL.

The multifunctional molecular complexes containing nucleic acid compositions according to the present invention may advantageously comprise generally from about 1 nanogram to about 1000 micrograms of DNA. In some desirable embodiments, the complexes contain 10 nanograms to 800 micrograms of DNA, inclusive. In more desirable embodiments, the complexes contain 0.1 to 500 micrograms of DNA, inclusive. In still more desirable embodiments, the complexes contain 1 to 350 micrograms of DNA, inclusive. In yet more desirable embodiments, the complexes contain 25 to 250 micrograms of DNA, inclusive. In the most desirable embodiments, the complexes contain about 100 micrograms DNA. Desirable methods of administration are intraperitoneal, intravenous, intramuscular, intradermal, subcutaneous, and inhalation. The complexes can also be delivered orally when used in combinations with other formulations changes, or when used in an emulsion.

The multifunctional molecular complexes containing nucleic acid compositions according to the present invention are formulated according to the mode of administration to be used. One having ordinary skill in the art can readily formulate a pharmaceutical composition that comprises a nucleic acid composition using the methods described herein. In cases where intramuscular injection is the chosen mode of administration, an isotonic formulation is desirably used. Generally, additives for isotonicity include sodium chloride, dextrose, mannitol, sorbitol, and/or lactose. In some cases, isotonic solutions such as phosphate buffered saline are desirable, Exemplary stabilizers include gelatin and albumin. The pharmaceutical preparations according to the present invention are prepared so as to be sterile and pyrogen free.

In accordance with the present invention there are provided pharmaceutical compositions which facilitate delivery of the multifunctional molecular complex, which in turn functions to facilitate transfer of the nucleic acid composition which is contained therein to the target cells. The pharmaceutical composition may be nothing more than an inert diluent and a pharmaceutically acceptable salt or ester form of the molecular complex. However, other pharmaceutically acceptable carriers well known to the artisan in this field can also be suitably employed to provide desired properties. Thus, one or more agents may be selected from the following recognized pharmaceutical classes of excipients: solvents; solvent systems; and solubilizing and dispersing agents including surfactants and emulsifying agents; viscosity modifying agents; and stabilizing and preservative agents, including antioxidants, WV absorbing agents, antibacterial agents, and buffering agents (see, e.g., the teaching of Remington's Pharmaceutical Sciences, 19th Edition, 1995, Gennaro (ed.) a standard text in the field).
The compositions and methods of the present invention are useful in the fields of both human and veterinary medicine. Accordingly, the present invention relates to RNAi, genetic immunization, and other nucleic acid-based therapeutic treatment of mammals, birds, and fish. The methods of the present invention can be particularly useful for such therapeutic treatment of mammalian species including human, bovine, ovine, porcine, equine, canine, and feline species.

In one aspect of the invention is provided a self-assembling delivery system for the transfer of a nucleic acid composition to a target cell comprising the following separate components capable of being brought together and self-assembling into a molecular complex by simple mixing: (i) one or more nucleic acids to be transferred; and (ii) two different cationic polyamine components each capable of binding to the nucleic acid, wherein the two cationic polyamine components are a targeting spermine and an endosomolytic spermine. The targeting spermine has a targeting ligand bound to a nitrogen atom of the spermine through a suitable linker group. In a desirable embodiment, the targeting ligand is bound to a secondary nitrogen atom. The targeting ligand may be, e.g., folic acid, folinic acid, 5-methyltetrahydrofolate, D-biotin, mannose, alpha-3'-propionyl thiomannoside, alpha-3'-propionyl propionyl thiomannoside-6-phosphate; lactose, or an antibody which binds specifically to a cell membrane protein. The endosomolytic spermine has a endosomal disruption promoting component, desirably a cholesteryl or fatty acid moiety, bound to a nitrogen atom of the spermine through a suitable linker group. In a desirable embodiment, the endosomal disruption promoting component is bound to a secondary nitrogen atom. The targeting ligand and the endosome membrane disruption promoting component are each be attached to its respective polyamine through a suitable linker group, e.g., through a PEG spacer arm or through an alkyl, carboxamide, carbamate, thiocarbamate, or carbamoyl bridging group. The endosomolytic molecule and the targeting molecules are each desirably attached though a selected linker to the secondary nitrogen of a spermine molecule.

### Examples of synthesis of the targeting ligand and endosome disrupting spermines

### Preparation of Bis-mannosyl spermine

The following is the synthesis of "bis- mannosyl spermine", or N⁴, N⁸- bis (5-N-(α-3'-propionamido thiomannoside) pentyl)-spermine tetraacetate. This is the method that was used to prepare the material for initial studies as a targeting agent for transfection of macrophages.

Subsequent experience preparing other polyamine based transfecting agents has resulted in alternative methods that improve the yields or reduce the number of steps needed to prepare similar compounds.

### 1) Preparation of N'-(4-(N'-hexahydropyrimidyl) butyl)-hexahydro-pyrimidine

A solution of spermine (23.8 g) in water (250 mL) was treated with a 37% v/v formaldehyde solution (18.7 g , or 6.9 g formaldehyde) and stirred 20 hours at room temperature. A small portion was extracted into chloroform, and the solvent was removed *in vacuo* to afford a wax (mass not determined). This small portion was used to confirm the structure of product by NMR. The remaining aqueous solution was taken directly on to the next step.

### 2) Preparation of N¹- (4- (N³ -tert-butyloxycarbonyl- N¹ - hexahydro-pyrimidinyl)-N³ - tert-butyloxycarbonyl-hexahydropyrimidine

The aqueous solution of N'-(4-(N'-hexahydropyrimidyl) butyl)-hexahydropyrimidine from the previous step (assuming 26.7 g) was treated with tetrahydrofuran (125 mL), N,N-diisopropylethylamine (62 mL), and di-tertbutyldicarbonate (56.5 g) for 22 hours at room temperature. The THF was removed, and the aqueous mixture was extracted into chloroform. The solvent was removed *in vacuo* to give an oil. The oil was purified on silica gel, eluting with a gradient of methanol in chloroform. Fractions containing the product (TLC R_{f} 0.58, CHCl₃ / methanol 9:1) were combined, and the solvent was removed to give 30.1 g of N¹-(4- (N³ - tert-butyloxycarbonyl- N¹- hexahydropyrimidyl)-N³ - tert-butyloxycarbonyl-hexahydropyrimidine as a wax.

### 3) Preparation of N¹, N ¹² -bis (tert-butyloxycarbonyl)spermine

N¹- (4- (N³ - tert-butyloxycarbonyl- N¹ - hexahydropyrimidyl)-N³ - tert-butyloxycarbonyl-hexahydropyrimidine (30.1 g) in ethanol (250 mL) was treated with pyridine (46 mL) and masonic acid (44.05 g) and refluxed for 5 hours. The solvent was removed *in vacuo,* and the residue was diluted with water (pH 5) and washed with chloroform. The aqueous layer was adjusted to pH 12 and extracted into chloroform. The solvent was removed to afford N¹, N¹² - bis (tert-butyloxycarbonyl) spermine (23.1 g) as a powder.

### 4 Preparation of N-CBZ-5-amino-1-pentanol

A solution of 5-amino-1-pentanol (20.07 g), and N,N-diisopropylethylamine (75 mL) in 300 mL of tetrahydrofuran was treated with benzyl chloroformate (31ml) for 1.5 hours at 0°C and 18 hours at room temperature. The solvent was removed *in vacuo* and the residue was extracted into chloroform. The chloroform was removed *in vacuo* for a wax which was crystallized from ethyl acetate and hexanes to give N-CBZ-5-amino-1-pentanol (26.2 g) as a crystalline solid.

### 5) Preparation of N-CBZ-5-amino-1-bromoentane

A solution of triphenylphosphine (11.05g), lithium bromide (10.98 g) and bromine (2.2 mL) in methylene chloride at 0°C was treated dropwise with a solution of N-CBZ-5-amino-1-pentanol (10.0 g) and N,N-diisopropylethylamine (17 mL) in methylene chloride. After stirring 20 hours at room temperature, the mixture was washed with water and extracted into methylene chloride. The solvent was removed *in vacuo* for a wax. The wax was purified on silica gel, eluting with a gradient of ethyl acetate in hexanes. Fractions containing product (TLC R_{f} 0.94, EtOAc/ Hex 1:1) were combined, and the solvent was removed to afford 7.23 g of N-CBZ-5-amino-1-bromopentane as an oil.

### 6) Preparation of N⁴, N⁸- bis (N -CBZ- 5- aminopentyl) - N¹, N¹² - bis (tert-butyloxycarbonyl) spermine

N¹,N¹²- bis (tert-butyloxycarbonyl) spermine (2.0 g) in 30 mL of acetonitrile was treated with N-CBZ-5-amino-1-bromopentane (compound 2; 1.49 g) and refluxed for 3 hours. The solvent was removed for an oil that was purified on silica gel eluting with a gradient of methanol in chloroform containing N,N-diisopropylethylamine (0.2%). Fractions containing the product (TLC R_{f} 0.35, CHCl₃ / methanol 9:1 + 0.4% DIEA) were combined, and the solvent was removed, to afford product (1.41 g) as an oil.

### 7) Preparation of N⁴, N⁸ - bis (5- aminopentyl),-N¹² - bis (tert-butyloxycarbonyl) spermine

N⁴, N⁸- bis (N-CBZ-5'-aminopentyl)-N¹, N¹² -bis(tert-butyloxycarbonyl) spermine (2.04g) was dissolved in 50 mL of methanol and treated with 2.04g of 20% Pd/C and 50 PSIG H₂ for 3.5 hours at room temperature. The Pd/C was removed by filtration through diatomaceous earth, and the solvent was removed from the filtrate *in vacuo* to give N⁴, N⁸- bis (5'-aminopentyl)-N¹, N¹² -bis(tert-butyloxy- carbonyl) spermine (0.79g) as an oil.

### 8) Preparation of mannose pentaacetate

A solution of D- mannose (20 g) in pyridine (75 mL) was treated with acetic anhydride (75 mL) at room temperature for 20 hours. Most of the pyridine and acetic anhydride were removed *in vacuo.* The residue was dissolved in chloroform and washed with water. Removal of solvent gave an oil/ taffy, which was purified on silica gel eluting with a gradient of 2- propanol in chloroform. Fractions containing the product (TLC R_{f} 0.7, CHCl₃/2- propanol 9: 1) were combined, and the solvent was removed to afford mannose pentaacetate (31.7 g) as a stiff oil.

### 9) Preparation of acetobromomannose

A solution of mannose pentaacetate (19.0 g) in 75 mL of methylene chloride at 0° C was treated with 30% HBr in acetic acid (75 mL) for 1.5 hours. The solution was extracted directly into chloroform and washed with saturated sodium bicarbonate solution. The solvent was removed *in vacuo* to give the product (18.0 g) as an oil.

### 10) Preparation of S-α-3'-propionyl tetra-O-acetyl thiomannoside

A solution of acetobromomannose (18.0 g) and thiourea (3.33 g) in acetone (200 mL) was refluxed for 3 hours. Water (200 mL), 3-iodopropanoic acid (9.63 g), anhydrous potassium carbonate (20 g), and potassium metabisulfite (9.73 g) were added and stirred for 4 hours at room temperature. The solution was diluted to 700 mL with water and washed with chloroform. The aqueous layer was acidified to pH 2 with 37% HCl and extracted into chloroform. The crude oil remaining after removal of the solvent was purified on silica gel, eluting with a gradient of ethyl acetate in hexanes. Fractions containing product (TLC R_{f} 0.36, EtOAc/ hex 1:2) were combined, and the solvent was removed to afford the product as a glass.

### 11) Preparation of Succinimid S-α- 3'-propionyl tetra-O-acetyl thiomannoside

A solution of S-α-3'-propionyl tetra-O-acetyl thiomannoside (2.94 g) in tetrahydrofuran (100 mL) was treated with N-hydroxysuccinimide (0.78 g) and N,N'-dicyclohexylcarbodiimide (1.39 g). The solution was stirred at room temperature for 20 hours, and then stored at 4° for 0.5 hour. A precipitate was filtered off, and the solvent was removed from the filtrate *in vacuo* to give succinimidyl S-α- 3'-propionyl tetra-O-acetyl-thiornannoside (3.80 g) as a white solid.

### 12 Preparation of N⁴, N⁸- bis (5-N-(α-3'-propionamido tetra-O-aeptyl thiomannoside) pentyl)- N¹, N¹² -bis(tert-butyloxycarbonyl) spermine

A solution of N⁴, N⁸- bis (5'-aminopentyl)-N¹, N¹² -bis(tert-butyloxycarbonyl) spermine (0.79g) in tetrahydrofuran (75 mL) was treated with N,N-diisopropylethylamine (0.64 mL) and succinimidyl S-α- 3'-propionyl tetra-O-acetyl-thiomannoside (1.32 g), and the solution was stirred at room temperature for 24 hours. The solvent was removed *in vacuo* to give a glass. The glass was purified on silica gel, eluting with a gradient of methanol in chloroform containing N,N-diisopropylethylamine (0.2%). Fractions containing the product (TLC R_{f} 0.30, CHCl₃ / methanol 9:1 ± 0.4% DIEA) were combined, and the solvent was removed to afford N⁴, N⁸- bis (5-N-((α-3'-propionamido tetra-O-acctyl thiomannoside) pentyl)- N¹, N¹² -bis(tert-butyloxycarbonyl) spermine- (0.69 g) as a glass.

### 13) Preparation of N⁴, N⁸- bis 5-N-α-3'-proionamido tetra-O-acetyl thiomannoside) pentyl) spermine tetraacetate

N⁴, N⁸- bis (5-N-(α-3'-propionamido tetra-O-acetyl thiomannoside) pentyl)-N¹, N¹²-bis(tert-butyloxycarbonyl) spermine (0.69 g) was dissolved in trifluoroacetic acid (20 mL) and stirred at room temperature for 1 hour. The solvent was removed *in vacuo,* the residue was dissolved in chloroform, and the solvent was removed (2X20 mL) to give 0.78 g of N⁴, N⁸- bis (5-N-(α-3'-propionamido tetra-O-acetyl thiomannoside) pentyl) spermine tetraacetate as an oil.

### 14) Preparation of N⁴, N⁸- bis (5-N-(α-3'-propionamido thiomannoside) pentyl)-spermine tetraacetate

N⁴, N⁸- bis (5-N-(α-3'-propionamido tetra-O-acetyl thiomannoside) pentyl) spermine tetraacetate (0.78 g) was dissolved in 25 mL of methanol, and 25 mL water and sodium carbonate (1.56 g) was added. The solution was stirred at room temperature for 5 hours. The solvents were removed *in vacuo,* and the residue was dissolved in 6 mL of 1% acetic acid and purified in three 2 mL aliquots on three Sephadex ^{™} G-25 medium columns (12 mL each), eluting with 1% acetic acid. Fractions containing the product were combined and lyophilized to give 0.31 g of N⁴, N⁸- bis (5-N-(α-3'-propionamido thiomannoside) pentyl)-spermine tetraacetate as a white solid. NMR (D₂O) δ 1.42 (m,4H), 1.59 (m, 4H), 1.79 (m, 9.5H), 1.95 (s, 38.5H), 2.14 (m, 5H), 2.62 (t, 4H), 2.94 (m, 4H), 3.10 (m, 4.5H), 3.24 (m, 18H), 3.72 (m, 6H), 4.05 (m, 6.5H), 5.34 (s, 2H).

### Alternate Method for Synthesis of N⁴, N⁸- bis (5-N-((-3'-propionamido thiomannoside) pentyl)-spermine hydrochloride ("bis-mannosyl spermine")

D-mannose was acetylated using acetic anhydride in pyridine to form mannose pentaacetate. The anomeric acetate was converted to the bromide with HBr/acetic acid in methylene chloride at 0 ° C to yield tetraacetobromomannose. The bromide was refluxed in acetone with thiourea followed by reaction with water, 3-iodopropionic acid, potassium carbonate, and potassium metabisulfite to afford S-(-3'-propionyl tetra-O-acetyl thiomannoside after acidification. This product was easily converted to the succinimide ester using N-hydroxysucainimide and N,N'-dicyclohexylcarbodiimide in tetrahydrofuran. Reaction with 5-amino-1-pentanol in tetrahydrofuran containing DIEA yielded the tetraacetomannosyl amide derivative of the alcohol. This alcohol was converted to the corresponding bromide as described above (triphenylphosphine, bromine, lithium bromide). Reaction with bis-BOC spermine (see above procedure) afforded both mono- and bis-derivatized (protected) mannosyl spermines. These spermines were separated by silica flash chromatography using a gradient of methanol in chloroform containing 0.2% DIEA (N,N-diisopropylethylamine). BOC groups were removed using 4N HCl in dioxane. The acetyl protecting groups were removed using sodium carbonate in methanol and water, pH 12. The product was obtained in overall 9% yield and was characterized by proton NMR (300 MHz FT-NMR), FAB mass spectroscopy, elemental analysis and thin layer chromatography. The penultimate product was characterized by C-18 HPLC using a gradient of acetonitrile in 0.1% TFA/water.

### Preparation of Trilactosyl spermine

N⁴-(5-(trilactosyllysyllysyl) amidopentyl spermine triacetate or "trilactosyl spermine" was prepared similarly to bis-mannosyl spermine. As with the latter compound, the method used to prepare trilactosyl spermine is described in full, along with suggested changes that have improved yields and/or reduced the number of steps in preparation of similar compounds.

### 1 Preparation of N¹-N⁴- methylenespermine

A solution of spermine (20.29 g) in water (50 mL) was treated with a 40% v/v formaldehyde solution (10.28 g) and stirred for 24 hours. The mixture was extracted into chloroform, and the solvent was removed to give the product (15.55 g) as a wax.

### 2) Preparation of N¹_N⁸- bis (tert-butyloxycarponyl) - N¹-N⁴ -methylene-spermidine

A solution of N¹-N⁴- methylenespermine (15.55 g) and N,N-diisopropylethylamine (52 mL) in tetrahydrofuran (300 mL) was treated with di-tert-butyldicarbonate (48.10 g) and stirred 3.5 days at room temperature. The solvent was removed *in vacuo,* and the residue was dissolved in ethyl acetate. This solution was washed with 5% NaOH solution, and then water. The solvent was removed *in vacuo* for an oil. The oil was purified on silica gel, eluting with a gradient of ethyl acetate in hexanes containing N,N- diisopropylethylamine (0.2%). Fractions containing the product (TLC R_{f} 0.17, EtOAc / hexanes 2:3 + 0.2% DIEA) were combined, and the solvent was removed to afford the product (22.06 g) as an oil.

### 3) Preparation of N¹-N⁸- bis (tert-butyloxycarbonyl)- spermine

A solution of N¹-N⁸ - bis (tert-butyloxycarbonyl) - N¹-N⁴-methylenespermine (18.28 g) in ethanol (300 mL) was treated with N,N-diisopropylethylamine (12.82 mL) and malonic acid (19.69 g). The mixture was refluxed 24 hours, and the solvent was removed *in vacuo.* The residue was diluted with water (pH was 4) and was washed with methylene chloride. The pH of the aqueous layer was adjusted to 9 with sodium bicarbonate, and extracted into methylene chloride. Removal of solvent gave a wax which was purified on silica gel, eluting with a gradient of 2-propanol in chloroform containing N,N-diisopropylethylamine (0.2%). Fractions containing the product (TLC R_{f} 0.21, 2-propanol / chloroform 3:7+0.25% DIEA) were combined and the solvent removed to afford the product (5.60 g) as a wax.

### 4 Preparation of N⁴-(4-cyanobutyl)- N¹-N⁸- bis (tert-butyloxycarbopyl)-spermine

A solution of N¹-N⁸- bis (tert-butyloxycarbonyl) - spermine (5.60 g), N,N-diisopropylethylamine (10.2 mL), potassium iodide (8.07 g), and 5-chlorovaleronitrile (5.472 mL) in acetonitrile (300 mL) was refluxed for 20 hours. The solvent was removed, and the residue was dissolved in chloroform and washed with water. The solvent was removed *in vacuo* for an oil. The oil was purified on silica gel, eluting with a gradient of ethyl acetate in hexanes containing N,N- diisopropylethylamine (0.4%). Fractions containing the product (TLC R_{f} 0.20, ethyl acetate / hexanes 4:1 + 0.4% DIEA) were combined, and the solvent was removed to afford the product (4.42 g) as an oil.

### 5) Preparation of N⁴- (5-aminopentyl)- N¹-N⁸- bis (tert-butyloxcarbonyl)-spermine

N⁴- (4- cyanobutyl)- N¹-N⁸- bis (tert-butyloxycarbonyl) - spermine (0.77 g) was dissolved in acetic acid and treated with 0.1 g of 5% Pd/C and 50 PSIG H₂ for 2.75 hours at room temperature. The Pd/C was removed by filtration through diatomaceous earth, and the solvent was removed from the filtrate *in vacuo* to give an oil. The oil was purified on silica gel, eluting with a gradient of methanol in chloroform containing N,N- diisopropylethylamine (0.4%). Fractions containing the product (TLC R_{f} at origin, methanol / chloroform 3:7 + 0.4% DIEA) were combined, and the solvent was removed to afford the product (0.32 g) as an oil.

### 6) Preparation of bis (N-α,ε-t-BOC) lysyl-N-ε-t-BOC lysine

A solution of BOC-Lys(BOC)ONp (10.0 g), N-e-t-BOC-L-Lysine (5.27 g), and N,N-diisopropylethylamine (13 mL) in dimethylformamide (400 mL) and water (100 mL) was stirred at room temperature for 48 hours. The solvents were removed *in vacuo,* and the residue was dissolved in chloroform. This solution was washed with dilute solvent HCl and dried, and the solvent was removed for an oil. The oil was purified on silica gel, eluting with a gradient of 2-propanol in chloroform containing acetic acid (0.5%). Fractions containing the product (TLC R_{f} 0.23, 2-propanol / chloroform 1:19 + 0.5% HOAc) were combined, and the solvent was removed to afford bis (N-α,ε-t-BOC) lysyl-N-ε-t-BOC lysine (13.1 g) as an oil.

### 7) Preparation of L-lysyl-L-lysine

Bis (N-α,ε-t-BOC) lysyl-N-ε-t-BOC lysine (12.2 g) was treated with trifluoroacetic acid (20 mL) and stirred at room temperature for 1 hour. The solvent was removed *in vacuo,* the residue was dissolved in chloroform, and the solvent was removed (3x20 mL) to give L-lysyl-L-lysine acetate salt (12.09 g) as an oil. The oil was dissolved in 0.1 N HCl and lyophilized to give L-lysyl-L-lysine hydrochloride salt (2.88 g) as a solid.

### 8) Preparation of lactose octaacetate

This intermediate was prepared similarly to mannose pentaacetate (compound 8 in the bis mannosyl spermine description). The product was a stiff oil.

### 9) Preparation of acetobromolactose

This intermediate was prepared similarly to acetobromomannose (compound 9 in the bis-mannosyl spermine description). The crude glass was recrystallized from ethyl acetate/ hexanes to give product as a solid. This compound must be stored frozen, as it is unstable at room temperature.

### 10) Preparation of S-(3'-propionic acid) thio hepta-O-acetyllactose

This intermediate was prepared similarly to S-α-3'-propionyl tetra-O-acetyl thiomannoside (compound 10 in the bis mannosyl spermine description). The product was found at TLC R_{f} 0.12 EtOAc / hexanes 1:1 + 0.7% HOAc as a white solid. In addition to TLC, NMR, elemental analysis, and FAB mass spec, this material was characterized using reverse phase HPLC. A YMC-Pack ODS-A, A 303-10 column (250 X 4.6 mm ID) eluted with a linear gradient of 100% A to 100% B (where A = 0.1% TFA in water, B = 0.1% TFA, 80% CH₃CN) at 2 mL/min over 20 minutes. Detection was conducted by using a PDA detector at 200-300nm. The product was found at 14.5 minutes.

### 11) Preparation of S- (Succinimidyl 3'-propionyl) thiohepta-O-acetyllactose

This intermediate was prepared similarly to succinimidyl S-α-3'-propionyl tetra-O-acetyl thiomannoside (compound 11 in the bis-mannosyl spermine description). The product was recrystallized from 2-propanol for a white solid. See the reverse phase HPLC conditions in compound 10 description. The product found at 15.4 minutes.

### 12 Preparation of tri-(acetyllactosyl) lysyllysine

L-lysyl-L-lysine hydrochloride (compound 7; 0.48 g) and N,N-diisopropylethylamine (0.654 mL) were dissolved in a 1:1 mixture of acetonitrile/water (150 mL) for a pH of 9. The solution was treated with S- (Succinimidyl 3'-propionyl) thiohepta-O-acetyllactose (compound 11; 3.60 g), which caused the pH to drop. The pH was monitored, and additional N,N-diisopropylethylamine was added as needed to maintain a pH of 8-9. After 24 hours at room temperature, the acetonitrile was removed *in vacuo,* and the aqueous solution was adjusted to pH 5 with dilute HCl. The mixture was extracted into chloroform, dried, and stripped of solvent for a glass. The glass was purified on silica gel, eluting with a gradient of 2-propanol in chloroform containing acetic acid (1%). Fractions containing the product (TLC R_{f} 0.25, 2-propanal / chloroform 1:9 +1% HOAc) were combined, and the solvent was removed to afford the product (0.76 g) as a glass. Reverse phase HPLC used a methods development column: YMC PACK ODS-A, A313-10, (6.0 X 250 mm ID) eluted with a linear gradient of 90% A to 100% B (where A = 0.1% TFA in water, B = 0.1% TFA, 80% CH₃CN) at 2 mL/min over 20 minutes. Detection by PDA detector at 200-300nm. The product was found at 20.4 minutes.

### 13) Preparation of Succinimidyl- tri -(acetyllactosyl) lysyllysine

Tri-(acetyllactosyl) lysyllysine (1.0 g) was dissolved in a 1:1 mixture of 2-prapanol/chloroform (20 mL) and treated with N-hydroxysuccinimide (48.1 mg) and dicyclohexylcarbodiimide (86.2 mg). The solution was stirred at room temperature for 19 hours, and then stored at 4° for 3 hours. A precipitate was filtered off, and the solvent was removed from the filtrate *in vacuo.* The residue was recrystallized from 2-propanol to give the product (0.76 g) as a white solid. See reverse phase HPLC conditions in compound 10 description; the product was found at 17.2 minutes.

### 14) Preparation of N⁴-(5-(acetylated tri-lactosyllysyllysyl)amidopentyl -N¹, N⁸-bis (tert-butyloxycarbonyl) spermine

To a solution of N⁴- (5-aminopentyl)- N¹-N⁸- bis (tert-butyloxycarbonyl) - spermine (compound 5; 87 mg) in methylene chloride (20 mL) was added a solution of succinimidyl- tri -(acetyllactosyl) lysyllysine (compound 13; 0.5 g) in methylene chloride. The solution stirred at room temperature for 18 hours, and the solvent was removed *in vacuo* for a solid. The solid was purified on silica gel, eluting with a gradient of 2-propanol in chloroform. Fractions containing the product (TLC R_{f} 0.16, 2-propanol / chloroform 1:4 +0.5% HOAc) were combined, and the solvent was removed to afford the product (0.38 g) as a white solid. See the reverse phase HPLC conditions in compound 10 description; the product was found at 18.1 min.

### 15) Preparation of N⁴-(5-(acetylated tri-lactosyllysyllysyl)amidopentyl spermine trifluoroacetate salt

N⁴-(5-(acetylated tri-lactosyllysyllysyl) amidopentyl -N¹, N⁸-bis (tert-butyloxycarbonyl) spermine (compound 14; 0.17 g) was dissolved in trifluoroacetic acid (5 mL) and stirred at room temperature for 2.5 hours The solvent was removed in vacuo and the residue dissolved in chloroform and the solvent removed (4x20 mL) to give 0.19 g of product as a sticky solid. See reverse phase HPLC conditions in compound 10 description; product found at 15.8 min.

### 16) Preparation of N⁴-(5-(trilactosyllysyllysyl)amidopentyl spermine triacetate

N⁴-(5-(acetylated tri-lactosyllysyllysyl) amidopentyl spermine trifluoroacetate salt (compound 15; 0.17 g) was dissolved in methanol and to the solution was added water (40 mL) and sodium carbonate (0.37 g). After stirring at room temperature for 4 hr, the solvents were removed *in vacuo* and the residue taken up in 3 mL of 1% acetic acid. The solution was purified on a Sephadex ^{™} G-25 medium column (16 mL), eluting with 1% acetic acid. Fractions containing the product were combined and lyophilized to give 62.7 mg of N⁴-(5-(trilactosyllysyllysyl) amidopentyl spermine triacetate as a sticky solid (extremely hydroscopie).

### Low pH Detergents (LPHD) for Endosomal Escape Function

The mechanism of transmembrane passage of cationic amphiphiles complexed with DNA is not well understood, but is believed to involve some combination of passive cell membrane disruption and active endocytosis by the cell. The latter mechanism involves eventual progression of the endosome to a lysosome with a concomitant decrease in pH from approximately 7.2 to 5.0. Degradative lysosomal enzymes are activated by the dropping pH, becoming maximally active near pH 5.0. Several endosome disrupting agents have been used to facilitate diffusion of the DNA through the endosomal membrane to minimize the damage to the DNA resulting from both the low pH and the lysosomal enzymes. We have developed a class of molecules which acquire a detergent like activity at low pH, but not at high pH. These "low pH detergents" (LPHD) provide lipophilic properties at pH 5-6. By ionically binding to and neutralizing DNA's negatively charged phosphodiester backbone, the complex as a whole is also rendered more lipophilic. Another attribute is that non-covalent, ionic binding allows multiple copies of the LPHD to be attached to a single molecule of DNA, providing the bulk property needed for insertion into the endosomal membrane lipid bilayer.

Compounds that are negatively charged (hydrophilic) at physiological pH and become neutral (lipophilic) as they are protonated with decreasing pH provide the detergent-like property. The pKa of these types of compound is the pH at which 50% of the molecules are ionized (unprotonated). At one pH unit below the pKa, only 10% of the molecules would remain ionized, whereas 90% would be protonated and detergent-like in nature. Thus, compounds of this type with a pKa of 6 or greater is significantly lipophilic and membrane disrupting at pH 5-6. Various polyamine derivatives with an appended carboxylic acid have pKa's that fall within the appropriate range.

### Preparation of "Low pH Detergent" (LPHD)

The following is the synthesis of a "Low pH Detergent" (LPHD) or N⁴, N⁹-bis (12'-dodecanoic acid)- spermine tetrahydrochloride.

### 1) Preparation of N'-(4-(N'-hexahydropyrimidyl)butyl)-hexahydro-pyrimidine

This is the same compound as intermediate (1) of the bis-niannosyl spermine synthesis.

### 2) Preparation of N¹-(4-(N³ -tert-butyloxycarbonyl-N¹-hexahydro-pyrimidinyl) -N³-tert-butyloxycarbonyl-hexahydropyrimidine

This is the same compound as intermediate (2) of the bis-mannosyl spermine synthesis.

### 3) Preparation of N¹, N ¹² - bis (tert-butyloxycarbonyl) spermine

This is the same compound as intermediate (3) of the bis-mannosyl spermine synthesis.

### 4) Preparation of benzyl 12-bromododecanoate

A solution of 12-bromododecanoic acid (5g), toluenesulfonic acid (0.5 g), and 3.73 mL of benzyl alcohol in toluene (100 mL) was distilled to near dryness and replenished with fresh toluene (100 mL), and the distillation was repeated. The residual toluene was removed *in vacuo,* and the crude was dissolved in ethyl acetate and washed with saturated sodium bicarbonate solution. The organic layer was dried, and the solvent was removed to afford an impure oil (8.61g) as a mixture of product and unreacted benzyl alcohol. The mixture was not purified further.

### 5) Preparation of N⁴-(benzyl 12'-dodecanoyl)-N¹, N¹² - bis (tert-butyloxycarbonyl)spermine

A solution of N¹,N¹²- bis (tert-butyloxycarbonyl) spermine (2.0 g) in acetonitrile (100 mL) was treated with acetic acid (0.268 mL) and benzyl 12-bromododecanoate (6.66 g). The solution was refluxed for 3 hours, and then stirred at room temperature for 18 hours. The solvent was removed at the rotary evaporator, and the residue was taken up in chloroform and washed with sodium bicarbonate solution. The organic layer was dried, and the solvent was removed *in vacuo* for 10.11 g of crude. The crude was purified on silica gel, eluting with a gradient of methanol in chloroform containing N,N-diisopropylethylamine (0.2%). Fractions containing the product (TLC R_{f} 0.23, CHCl₃ / methanol 9:1 + 0.2% DIEA) were combined, and the solvent was removed to afford the product (0.62 g) as an oil.

### 6) preparation of N⁴-(12'-dodecanoic acid)-N¹, N¹²-bis(tert-butyloxycarbonyl) spermine

N⁴ - (benzyl 12'-dodecanoyl) N¹, N¹² - bis (tert-butylaxyoarbanyl) spermine (0.29 g) was dissolved in 30 mL of methanol and treated with 0.03 g of 10% Pd/C and 50 PSIG H₂ for 1.5 hours at room temperature. The Pd/C was removed by filtration through diatomaceous earth. The solvent was removed from the filtrate *in vacuo* to give N⁴ - (12'-dodecanoic acid) spermine tetrahydrochloride as an oil (0.19 g).

### 7) Preparation of N⁴ - (12'-dodecanoic acid) spermine tetrahydrochloride

N⁴ - (12'-dodecanoic acid) - N¹ , N¹² - bis (tert-butyloxycarbonyl) spermine (0.19 g) was dissolved in trifluoroacetic acid (10 mL) and stirred at room temperature for 1 hour. The solvent was removed *in vacuo,* the residue was dissolved in chloroform, and the solvent was removed (3 X 25 mL) to give an oil. The oil was dissolved in 0.1 N HCl and lyophilized to give 0.18g of N⁴-(12'-dodecanoic acid) spermine tetrahydrochloride. NMR (DMSO-d₆) δ 1.26 (m, 16.7H), 1.48 (m, 2.7H), 1.70, 1.78 (overlapping m, 6H), 2.01 (m,4H), 2.91 - 3.17 (overlapping m, 12H), 8.17 (br m, 5.3H), 9.30 (br m, 2H).+

### Synthesis of Cholestryl Spermine (CSm): Synthesis of N⁴- (5-N-(3β-O-carbamoyl-5-cholestene)-1-pentyl)-spermine hydrochloride ("cholesteryl spermine")

A hexahydropyrimidine cycloadduct of spermine and formaldehyde was formed, and the secondary amines were converted to the di-*tert*-bntoxycarbonyl (BOC) derivative using di-*tert*-butyl dicarbonate, (BOC)₂O in water and THF. The methylene bridge was removed (malonic acid, pyridine in ethanol) to afford the bis-BOC spermine intermediate as previously reported. Cholesteryl chloroformate was reacted with 5-amino-1-pentanol in methylene chloride with *N,N-diisopropylethylamine* (DIEA) to form the desired carbamate linkage. This alcohol was converted to the corresponding bromide using triphenyl phosphine, bromine, lithium bromide, and DIEA in cold methylene chloride. Nucleophilic attack of the cholesteryl bromide by bis-BOC spermine in the presence of potassium carbonate in methylene chloride resulted in both mono- and bis-substituted cholesteryl spermines. These were easily separated by silica flash chromatography using a gradient of methanol in chloroform containing 0.2% DIEA. Deprotection of BOC groups by 4N HCl in dioxane afforded the final mono-cholesteryl spermine product as the hydrochloride salt. The product was obtained in overall 16% yield and was characterized by proton NMR (300 MHz FT-NMR), FAB mass spectroscopy, elemental analysis, and thin layer chromatography.

### Example 1: Targeted Transfection of Mannose Preceptor Containing Immune Vells In Vivo

The following experiments were performed essentially as described below to demonstrate the ability of mannosyl spermine and cholesteryl spermine to facilitate the delivery of nucleic acids into animals.

### Preparation of complexes

DNA (GFP expression plasmid, EGFP, Clontech, CA) is mixed with a mixture of mannosyl spermine and cholesteryl spermine, synthesized as described above. Typically, 1 mL of a 2 mg /mL of DNA is mixed with 1 mL of 2 mg /mL, of spermine mixture. Spermine mixtures are prepared by mixing the two spermine molecules: the targeting spermine molecule (mannosyl, lactosyl, folate, or biotinylated spermine) with cholesteryl spermine. The molecular ratio is maintained at; e.g., 65% cholesteryl and 35% mannosyl, lactosyl, folate, or biotinylated spermine. When DNA and the spermine mixture are mixed together, a co-complex of DNA is formed with both modified spermine molecules. The charge ratio achieved at the concentrations indicated is approximately 0.8 (positive / negative). Although, the complexes contain both spermine molecules, they are heterogeneous, both in the distribution of particle charge and the ratios of bound spermine derivatives. The composition of the complexes reflects the concentrations, charge ratio, and the relative amounts of the two spermines. The solutions are mixed into the final citrate buffer (30 mM, pH 6.8) with 150 mM sodium chloride. Other complexes of DNA are prepared in which the ratio of positive to negative charges in the spermine-DNA solution mixture are varied from 0.5 to 1.2. Other ratios of the two spermines are also prepared in mixtures with DNA and compared against the 35% mannosyl, lactosyl, biotinylated, or folate targeting spermine - 65% cholesteryl spermine mixtures of DNA.

### In Vivo Studies

The mixtures are injected intraperitoneally (100 uL) into the peritoneal cavity of 5 mice for each formulation variant. At 5 days postinjection, when *in vivo* plasmid expression peaks, peritoneal fluid is removed. One hundred microliters of peritoneal fluid is placed on a slide and cytospotted by centrifugation at 3000 rpm. The slides are viewed by UV fluorescent microscopy to detect GFP fluorescence. A part of the peritoneal fluid is subjected to FLOW analysis (Beckman- Coulter) to quantitatively determine the amount of fluorescence and the number of cells transfected. Another part of the peritoneal fluid derived cells are stained with FITC or rhodamine conjugated MAC1 antibody (R&D Systems) and yet another peritoneal fluid fraction is stained with FITC or rhodamine labeled mannosylated BSA (bovine serum albumin)(Siigma Cemical Co. St. Louis, MI). Control mice included DNA free of added complexing agents and DNA complexed with either mannosyl spermine or cholesteryl spermine.
Mice injected with the formulation containing spermine to DNA at a charge ratio of 0.8 and at the ratio of mannosylated spermine to cholesteryl spermine 35% to 65% express EGFP at levels exceeding those expressed at other formulations. The results are similar when analyzed by FLOW analyses (Beckman Coulter, CA), and the majority of MAC1 positive cells (macrophages) are transfected. Almost 100% of mannose receptor containing cells expressed EGFP. Taken together the results indicate successful targeted transfection *in vivo* of immune cells, directed through DNA internalization using the mannose receptor. Furthermore, the use of cholesteryl spermine as an endosome / lysosome breaker has been shown to be effective. To further substantiate these conclusions, the cells were analyzed by confocal microscopy, and the results were consistent with the predictions. Only at the optimal formulation conditions the rhodamine labeled DNA (GT Systems, San Diego, CA) is in the nucleus, while the control suboptimal formulations used in the study at 10:90 and 90: 10 (targeting spermine to endosome disrupting spermine) result in DNA either being not targeted to the cells or trapped in the endosome /lysosome as determined from intracellular localization of rhodamine.
Similar results (transfection at 0.8 charge ratio and spermine ratio of mannosyl spermine to cholesteryl spermine of 35 to 65) are observed in cells derived from the spleen when the complexes are delivered intravenously, through tail vein injections. The complexes are prepared as described above except that 5 mM citrate and 150 mM NaCl are used as buffer. To determine the transfection efficiency, the spleen is removed from mice on day 5. Cells are recovered by crushing the spleen on a slide and observing EGFP fluorescence by fluorescent microscopy. The cell type is identified by cross-staining staining the preparation with labeled MACl antibody (macrophage identification) or labeled mannosylated BSA (identification of mannose receptor containing cells) (Current Protocols in Immunology). The cells are primarily of macrophage type and a majority (>95%) of the mannose receptor containing cells are transfected.

In order to observe transfection of Langerhans cells, biopsies of skin are taken on day 5 following subdermal and intradermal injections (10 uL of a Img /mL injection of complexes prepared as described previously). Histological analysis is performed by sectioning paraffin embedded tissue and observing fluorescent cells cross stained with labeled mannosylated BSA. The Langerhans cells are transfected maximally at the optimal ratio as indicated above.

### Example 2: Targeted Transfection of Hepatocytes In Vivo

Experiments similar to those described in Example 1 for mannosyl spermine and cholesteryl spermine may be used to test the ability of any other spermine-containing compounds to facilitate the delivery of nucleic acids into animals. Exemplary methods are described below.

### Preparation of Complexes

DNA (Beta galactosidase expression plasmid - Clontech, CA) is mixed with a mixture of lactosyl spermine (mono or trilactosylated) and cholesteryl spermine. Typically, 1 mL of a 2 mg /mL of DNA is mixed with 1 mL of 2 mg /mL of spermine mixture (approximate charge ratio of 0.8 positive to negative). Spermine mixtures are prepared by mixing the two spermine molecules, the targeting spermine molecule (lactosyl spermine) with the cholesteryl spermine. The molecular ratio is maintained at 65% cholesteryl and 35% lactosyl spermine. When DNA and the spermine mixture are mixed together a co-complex of DNA is formed with both modified spermine molecules. The charge ratio achieved at the concentrations indicated is approximately 0.8 (positive / negative). Although the complexes contain both spermine molecules, they are heterogeneous, both in the distribution of particle charge and the ratios of bound spermine derivatives. The composition of the complexes reflects the concentrations, charge ratio and the relative amounts of the two spermines. The solutions are mixed into the final citrate buffer (5 mM, pH 6.8) with 150 mM sodium chloride. Other complexes of DNA are prepared in which the ratio of positive to negative charges in the spermine-DNA solution mixture are varied from 0.5 to 1.2. Other ratios of the two spermines are also prepared in mixtures with DNA and compared against the 35% lactosyl - 65% cholesteryl spermine mixtures of DNA.

### In Vivo Studies

The mixture is injected intravenously (100 uL) of 5 mice for each formulation variant. Formulations variants included different charge ratios and various lactosyl spermine to cholesteryl spermine ratios. At 5 days postinjection, when *in vivo* plasmid expression peaks, the mice are sacrificed, and their livers are removed and snap frozen for histological analysis. Several slices of the liver tissue both near and farther from the major blood vessels and the bile ducts are embedded into paraffin and sectioned using a microtome. The tissue slices are stained using X-gal (an enzymatic substrate for betagalactosidase). The liver tissue slices are stained using labeled lactosylated BSA, as a cross stain to identify hepatocytes (asialoglycoprotein receptor). The slides are viewed under an UV fluroscent microscope to detect the label fluorescence, and the betgal stain is observed under light microscopy. Control mice are administered "naked" DNA free of added complexing agents and DNA complexed with either lactosyl spermine or cholesteryl spermine, but not both.

### Expected Results

Based on the results from Example 1, mice injected with the formulation containing spermine to DNA at a charge ratio of 0.8 and at the ratio of lactosylated spermine to cholesteryl spermine of 35% to 65% are expected to express beta-galactosidase at levels exceeding those expressed using a formulation of 10% lactosyl and 90% cholesteryl. The expression of beta-galactosidase in the hepatocytes in the liver tissue can be analyzed by fluorescent microscopy of tissue slices following fluorescently labeled lactosylated BSA staining. Expression of beta-galactosidase in hepatocytes is an indication of successful targeted transfection *in vivo* of hepatocytes, directed through DNA internalization using the asialoglycoprotein receptor, and successful use of cholesteryl spermine as an endosome / lysosome breaker. To further substantiate the conclusions, the cells can be analyzed by confocal microscopy.

### Example 3: Trilactos Spermine Complexes are Preferentially Taken Up by the Liver and Mono and Bis-Mannosyl Spermine Complexes Target Liver Cells through Transfection of Kupfer Cells

The following experiments were performed essentially as described below to demonstrate the,ability of trilactosyl spermine and mono and bis-mannosyl spermine to facilitate the delivery of nucleic acids.

### Labeling DNA

Bacteria containing the beta-galactosidase expression plasmid are grown in minimal M9 media (Miller, CSHS laboratory Press) in glucose as the sole carbon source overnight. The cells are washed in minimal media thrice through centrifugation and resuspension in M9 media without the carbon source, and resuspended in M9 with uniformally 14C-labeled ribose (NEN, MA) and deoxynucleosides (NEN, MA) for 3 hours. The cells are harvested, and the plasmid is isolated using Qiagen miniprep columns (Qiagen, Inc., CA). The labeled plasmid is mixed with unlabeled plasmid to achieve a specific activity of 3 million CPM per 100 ug plasmid.

### Complexing with Modified Spermines

DNA is complexed with spermines as described in the previous two examples at the optimal charge ratio of 0.8 with either the monolactosyl, trilactosyl, or mannosyl (mono and bis) spermine to achieve a final DNA concentration of 1.0 mg /mL in citrate buffer at mM and 150 mM NaCl.

### In Vivo Studies

The mice, 5 per group, are injected intravenously through the tail vein (100 uL). Mice are sacrificed at various times as indicated for serum analysis of plasmid DNA to determine blood clearance. To determine the amount of plasmid in blood, one mL of serum is placed in liquid scintillation counters with fluor to quantify the CPMs associated with each sample. In order to determine tissue distribution, various organs are harvested on day 5 (peak of expression from previous studies) and snap frozen. Whole organs from the animals are harvested livers from 3 animals in the group of 5 are ground in mild alkali (100 mM KOH) and neutralized with acid (HCl) prior to scintillation counting. Livers from the remaining two animals are embedded in paraffin and sectioned as described in Example 2 and stained for betagalactosidase activity, as described.

### Blood Clearance

In the study evaluating the distribution and blood clearance of DNA following an IV administration, labeled DNA is separately complexed with modified spermines (only monocomplexcs of any one slaez-mine is used in this study). Mannose (mono and bis) and lactose (mono and tri) spermines are used separately in these complexes. Unmodified spermine and spermidine complexes with DNA serve as controls. Consistent with the prediction, the fastest clearance is with trilactosyl spermine (extrapolation of the curve suggests uptake in a couple of minutes). Similarly, monolactosyl derivatives also clear the blood rapidly, but about 10 times more slowly than trilactosyl spermine complexes. Control complexes are not predicted to have any cell targeting features and therefore, they are present in the blood for much longer periods. The results demonstrate that in the absence of cell surface protein targeting the blood levels of plasmid DNA remain unchanged for several hours. Trilactosylated molecules have been previously shown to target hepatocytes when conjugated to proteins (covalent and non-covalent linked) and administered IV. Histological analyses addresses the specificity of cell targeting (Hepatocytes *vs.* Kupfer) in more detail. Bismannosyl spermine complexes also allow for rapid clearance of DNA from blood, but approximately 50 -100 times more slowly than trilactosyl spen-zaine complexes. In a separate study, bismannosyl spermine complexes are demonstrated to be taken up exclusively by cells of the macrophage and dendritic cell lineages (Kupfer, Langerhans, and splenic and lung macrophages - Example 1) following IV administration.

### Tissue Distribution

In the second part of the study, distribution of DNA amongst some of the major organs is evaluated 5 days following IV administration. A simple tissue grind and scintillation counting is carried out. The organs chosen (lung, heart, kidney, spleen, and liver) for analysis represent the major capillary beds. Over 95% of the DNA eventually taken up (previous studies using quantitative PCR) is found in these organs (excepting the heart) following IV administration. Of the administered DNA, a large majority is usually degraded and excreted (>90%) in 48 hours. However, with lactosylated and mannosylated spermine complexes; retention of DNA was between 30 and 85%, respectively. The total amount of CPMs in all of the organs combined vary considerably; trilactosyl > monolactosyl ≅ bismannosyl ≅ monomannosyl > >> controls.

Betagalactosidase activity staining of tissue sections indicates that administration of lactosyl spermine complexes results in DNA internalization of DNA exclusively into hepatocytes; however, expression is poor. This is attributed to the lack of endosome disrupting spermine (cholestryl) in the complex used in this study. Based on the results presented in Examples 1 and 2, it is inferred that co-complexes of both spermines are necessary to achieve optimal expression. The results described in this example are consistent with the hypothesis that spermines modified with the receptor binding ligand when complexed with DNA target specific cell types that contain the receptor and are internalized; however, expression of these internalized plasmid DNAs require additional biochemical functionalities such as endosomal / lysosomal membrane disrupting activities.

While the lactosyl spermine complexes primarily transfect hepatocytes following IV administration based on betagalactosidase activity staining, the mannosyl spermine complexes transfect Kupfer cells (macrophage lineage) based on betagal staining of the liver slices. The DNA distribution data is also suggestive of the ability of mannosyl spermine complexes to transfect alveolar macrophages in the lung and dendritic and macrophage cells in the spleen. It is therefore possible to generate formulations based on powder or aerosol based delivery systems to transfect lung (alveolar) macrophages using DNA complexes that contain mannosyl spermine using inhalation technologies (Becton-Dickinson, Inhale therapeutics and Powderject Vaccines).

### Example 4: Oral and Intravenous Deliver Studies In Vivo Using Microemulsions

The following experiments were performed essentially as described below to demonstrate the ability of microemulsions to facilitate the delivery of nucleic acids into animals.

The objective is to develop microemulsion (ME) formulations designed for oral delivery of DNA. Microemulsions for oral delivery are prepared using a mixture that contains DNA or other nucleic acids (2.0 mg / mL in, e.g., 30 mM citrate, 150 mM NaCl pH 6.8 buffer) and benzalkonium chloride (0.1%). The mixture is further mixed in equal volumes to a mixture that contains seven parts of Labrasol^{™} (Gattefosse, Saint-Priest Cedex, France), one part of Lauroglycol (polypropylene esters such as Lauroglycol FCC for oral administration from Gattefosse, France) and two parts of ethanol. Other formulations contain bupivacaine at 0.25%, octyl spermine at 2.0 mg / mL, or cholesteryl spermine at 2.0 mg / mL, instead of benzalkonium chloride and lauroglycol in the above formulation. Formulations that contain cationic amphiphiles such as benzalkonium chloride, octyl spermine, cholesteryl spermine and bupivacaine result in oil in water emulsions and the DNA tested is in the oil phase, because DNA complexes of cationic amphiphiles are hydrophobic. The DNA tested is an expression plasmid for luciferase (Promega Corp.). When all of the components are mixed, microemulsions are readily formed and are thermodynamically stable. The formulation (100 µL) containing DNA is fed to mice (5 in one formulation group) by intubating the stomach. Five days following administration, several organs including kidney, liver, lung, intestinal segments (ileum, duodenum, small intestine and large intestine), brain, heart, and spleen are harvested and snap frozen. The tissues are homogenized in luciferase buffer (Promega Corp., Madison, WI) using a polytron. Luciferase activity is determined using a luminometer (Berthold) and in the assay buffer (Promega Corp., Madison, WI). Luciferase activity is mostly detected in the duodenum, ileum, and small intestine for all formulations (>70%). A significant amount (∼10% of the total luciferase activity in the animal is detected in the liver. It appears that emulsions are picked up by the portal circulation from the GI tract and delivered to the liver. Maximal systemic absorption of DNA is observed when formulations contained bupivacaine, cholesteryl spermine, benzalkonium chloride, and octyl spermine.

For intravenous delivery of microemulsions, the mixtures were prepared similarly except that Labrasol was replaced with soybean oil. Other oils such as vegetable and fish oils (e.g. oils sold for consumption by humans) may also be used. Soybean oil MEs when delivered (20 uL / dose) results in transfection of all organs that have large capillary beds, such as the lung, liver, spleen, and kidney. These organs demonstrate high amounts of luciferase activity.

### Example 5: In Vivo Model for Gene Silencing using Plasmid DNA Complexed with Local Anesthetics to Determine the Utility of Local Anesthetic Complexed Nucleic Acids for RNAi Applications

### Background and Rationale

The invention also features compositions that include an anesthetic, e.g., bupivacaine and a dsRNA and methods for delivering a complex of an anesthetic and a dsRNA into a cell or an animal. Accordingly, the following experiments were performed to demonstrate the ability of bupivacaine to facilitate the delivery of dsRNA. The experiments were not designed to elicit post transcriptional gene silencing (PTGS) or RNAi *per se,* rather they were designed to follow the kinetics of DNA vector expression in mice in the absence of an immune response. The results indicate the presence of a non-immune mediated, gene-specific silencing mechanism suggestive of dsRNA mediated PTGS. Although the use of plasmid DNA based expression vectors represents an inefficient way of generating dsRNA, it none-the-less has been shown to give rise to sufficient levels of dsRNA required for PTGS induction in plants and in cell culture. This result and the observed presence of dsRNA species in animals injected with plasmid DNA are consistent with dsRNA mediated gene-silencing. The ability to generate dsRNA vanes for different plasmids and is determined in part by the presence of cryptic promoter elements. In the expression vectors used for these studies, silencing does not occur for several months while other vectors may invoke silencing faster and some not at all. In contrast, vectors specifically designed to generate dsRNA rapidly induced silencing.

In animal experiments designed to evaluate the kinetics of expression of transgenes encoding autologous proteins (for example, murine IL-12 or murine Factor VIII in mice), we had observed that transgene expression was not only transient, but that subsequent re-administration of that transgene did not rescue transgene expression, suggesting that transgene expression was silenced. An immune mechanism has been ruled out and a molecular mechanism is proposed.

### Silencing of IL12-p4O Eexpression in Balb/C mince

Expression of mouse IL-12 has been shown to be augmented over baseline expression levels upon intramuscular inoculation of mice with plasmids expressing murine IL-12. Expression of the murine IL-12 transgenes was achieved through the use of vectors containing the HCMV IE promoter and SV40 polyadenation signal. BLISA of serum IL-12 p40 levels affords a facile system to analyze expression levels and the effectiveness of the administered DNA. p40 is one of the two polypeptide chains comprising IL-12.

In all of the RNAi studies described here using IL12 DNA (IL12, dsRNA producing vector and vector encoding T7 RNA polymerase), ILI2 DNA was administered as a complex with bupivacaine, a local anesthetic, since higher *in vivo* transfection efficiencies (∼3X) are achieved with this complex over those mediated by naked DNA. Bupivacaine complexes were prepared by mixing 2.0 mg mL of DNA in Citrate buffer (30 mM), 0.1 % EDTA and 150 mM NaCl; pH 6.7, 5X,concentrated buffer ( 1X = Citrate buffer (30 mM), 0.1% EDTA and 150 mM NaCl; pH 6.7 ) and bupivacaine to yield a final bupivacaine concentration of 0.25% and buffer components at IX (Citrate buffer (30 mM), 0.1% EDTA and 150 nm4 NaCl; pH 6.7).

We observed that inoculation of 100 µg of IL12 plasmid induces significant serum levels of p40 (700-800 pg/mL). The expression peaks 5-8 days following inoculation, and then drops to baseline levels in about 140 days. We have shown that re-inoculation of the TL-12 plasmid at a time when IL-serumIL- IL-12 has dropped to near baseline levels (days 150 and 240) does not induce the anticipated serum levels of IL12. DNA saturation at the site of primary injection does not appear to play a role in the inability to re-express IL-12, as booster injections given in the hind leg other than the one used for primary injection are not able to reconstitute IL-12 expression. These results suggest that the there is a systemic block to expression as opposed to a local one. Systemic silencing mediated by PTGS is a hallmark of PTGS and has been observed in plants, *C. elegant* and zebrafish. Interestingly, a third inoculation (day 240) results in further reduced IL12 levels to below baseline levels, suggesting that the chromosomal copy may also have been silenced.

In another set of experiments, murine IL-12 p40 expression in sera was monitored in mice following intramuscular injection of a 100 ug dose of murine IL-12 expression plasmids or in naive mouse controls that were not injected. Average value from 5 mice were compared; less than 20% scatter was observed. Timepoints of primary and booster injections were at days 0, 150, and 240. The cutoff value for the p40 ELISA assay is about 100 pg/ml.

Murine IL-12 p40 serum expression was monitored following intramuscular injection of 100 ug IL-12 expression plasmids in mice injected at days 0 and 150 or in mice injected only at day 150. Average value from 5 mice were compared; less than 20% scatter was observed.

### Silencing in SCID Mice

Murine IL-12 p40 serum expression was monitored in SCID mice following intramuscular injection of 100 ug of murine IL-12 expression plasmids or in naïve SCID mouse controls that were not injected. At days 0 and 60, the mice received their primary and booster injections. Average value from 5 mince were compared; less than 20% scatter was observed. Silencing seems to occur faster in SCID mice.

Control mice that were age matched supported expression of the IL-12 transgene to predictable levels (700-800 pg/mL) at day 150, indicating that age does not play a role in the inability to express the IL-12 transgene. In addition, mice that received a primary inoculation of another transgene vector at day 0 potentiated IL12 expression to levels of 700-800 pg/mL when the IL-12 expression vector was administered at day 150. No detectable immune responses to IL12 were observed suggesting that anti-IL-12 responses do not play a role in loss of IL-12 expression. Furthermore, similar results obtained in SCID mice support the absence of an immune mediated mechanism and indicated the involvement of a molecular mechanism such as PTGS. Preliminary analyses of p40 RNA levels in tissue samples further support a PTGS mediated mechanism for the observed decrease in expression.

Silencing is not mediated by an IL-12 effect for the following reasons. Injection of the mice with 50 ug recombinant murine IL-12 at day 0 does not prevent subsequent expression of the murine IL-12 expression plasmids. In addition, experiments utilizing murine IL-12 RNA expression vectors in which IL-12 specific RNAs are made but in which no IL-12 protein is made, also result in down-regulation of IL-12 expression. During transcription the majority of transcripts are initiated at positions prescribed by promoter elements; however, transcripts also originate at other sites (cryptic promoter sequences) in the template DNA. Unlike specific RNA transcripts initiated through promoter elements, promiscuous transcript initiation results in a plurality of aberrant species. Some of the aberrant transcripts are derived from the non-template strand of DNA, resulting in the formation of antisense RNA, which in turn forms dsRNA species when abundant transcripts of the template strand are also available. Therefore; expression cassettes that contain highly active promoters such as the HCMV-IE are predicted to favor the formation of dsRNA. Mediation of PTGS by dsRNA has been shown to be catalytic, and the presence of non-stochiometric amounts of dsRNA has been shown to be effective in mediating the degradation of target mRNAs. Formation of gene specific dsRNA is particularly favored in plasmid based expression system, where the circularity of the plasmid molecule affords all aberrant transcripts initiated in the plasmid to read through the gene sequence, resulting in the generation of the dsRNA species. In experiments that utilized plasmid DNA for the expression of mouse IL12 p40, presence of aberrant (antisense) transcripts arising at 0.2% of p40 mRNA levels in both, transfected cells and in vivo in muscle tissue of mice intramuscularly inoculated with the expression plasmid. We have also demonstrated the presence of IL-12 specific dsRNA species in transfected mouse muscle.

In conclusion, silencing of transgene expression was induced by the transgene itself, in the absence of an immune response to the expressed protein. The phenomenon of silencing appears to be systemic, preventing any expression from subsequently administered plasmids, regardless of the site of re-administration.re-adminstration.re-administration. However, we do not know if the observed systemic effect is due to a true spreading of the silencing signal or whether it is instead a reflection of injected plasmid molecule bio-distribution. Bio-distribution studies indicate that the injected plasmid DNA is found in all tissues with the exception of brain and gonad.

### Pre-administration of IL-12 dsRNA or of an IL-12 dsRNA Expression Vector Silences IL-12 Expression in a Sequence Dependent Manner

At day 0, groups 3 and 4 received by intramuscular injection, IL-12 specific dsRNA or an IL-12 specific dsRNA expression vector. Control groups 1 and 4 received no injection at day 0. On day 5, groups 2, 3, and 4 were injected intramuscularly with an IL-12 expression vector and on day 13, all mice were bled and serum IL-12 levels measured. Control groups injected with HSVgD-2 RNA and HSVgD-2 dsRNA expression vectors had no effect on IL-12 expression.

The observations from this work are intriguing and suggestive of dsRNA mediated PTGS. In this event, we realize that the expression vectors used in this experiment are not optimally designed to make dsRNA. One system that enables the efficient transcription of dsRNA *in vitro* and *in vivo* is the T7 RNA polymerase transcription system. Therefore, to more strongly demonstrate that dsRNA is mediating the observed silencing effect in mice, mice were injected on day 0 with a T7 RNA polymerase expression vector and a dsRNA T7 expression vector that encodes either a 600bp IL-12 specific dsRNA or an irrelevant control 600bp dsRNA comprised of herpes simplex-2 glycoprotein D (HSVgD-2) specific sequences. Alternatively, mice were pre-injected with 600bp long IL-12 or HSVgD-2 specific *in vitro* transcribed naked dsRNA. The mice were then injected on day 5 with an IL-12 expression vector and serum IL-12 levels were measured on day 13. It is an important consideration for these experiments that all of the dsRNAs used in these experiments are unable to be translated into protein products. Therefore, any observed silencing cannot be attributed to an IL-12 mediated effect.

### Results and Conclusions

Sera from Group 1 show basal endogenous levels of IL-12, 200 pg/ml. Group 4 serum IL-12 levels of 800pg/ml represent total IL-12 comprised of both endogenous IL-12 and expression vector derived IL-12. Pre-administration of dsRNA (Group 2) results in decreased levels of IL-12 when compared to the group that did not receive RNA (Group 4); however, pre-administration of vectors that enable intracellular dsRNA synthesis (Group 3) results in the inability to detect any IL-12. Pre-administration of the control HSVgL7-2 dsRNA or dsRNA expression vector had no effect on IL-12 expression. These results demonstrate that Il-12 silencing is mediated both by injection of IL-12 specific dsRNA and by injection of an IL-12 specific dsRNA expression vector. Silencing is sequence specific as only the IL-12 specific dsRNA and IL-12 specific dsRNA expression vector had any effect upon IL-12 expression: the HSVgd-2 dsRNA and expression vector had no measurable effect upon IL-12 expression. In addition, these results demonstrate vector mediated intracellular expression of dsRNA is more effective than administration of dsRNA. However, it is possible that the full potential of the dsRNA effect in Group 2 was not realized under the delivery conditions employed here and that alternative methods of delivery and or dose potentially can be more effective. It is also possible that had we looked later than day 13, IL-12 levels in Group 2 may have gone down further. It is interesting to note that there was no detectable IL-12 in Group 3 at day 13, indicating that both endogenous and vector derived IL-12 expression was suppressed. Interferon responses to the injection of dsRNA and the dsRNA expression vectors were also monitored during the first week of the experiment. Injection of both the IL-12 and HSVgD-2 specific dsRNAs induced a significant interferon response that was rapidly induced but last for a short duration, 3 days. No detectable interferon response was seen in those animals injected with the dsRNA expression vectors. This result is consistent with the data generated in the cell culture models.

### Other Embodiments

From the foregoing description, it will be apparent that variations and modifications may be made to the invention described herein to adopt it to various usages and conditions. Such embodiments are also within the scope of the following claims.

All publications mentioned in this specification are herein incorporated by reference to the same extent as if each independent publication or patent application was specifically and individually indicated to be incorporated by reference.

Preferred Embodiments
1. A composition comprising a nucleic acid, an endosomolytic spermine that includes a cholesterol or fatty acid, and a targeting spermine that includes a ligand for a cell surface molecule, wherein the ratio of positive to negative charge of said composition is between 0.5 and 1.5, inclusive, wherein said endosomolytic spermine constitutes at least 20% of the spermine-containing molecules in said composition, and wherein said targeting spermine constitutes at least 10% of the spermine-containing molecules in said composition.
2. The composition of preferred embodiment 1, wherein the ratio of positive to negative charge is between 0.8 and 1.2, inclusive.
3. The composition of preferred embodiment 1, wherein said nucleic acid comprises DNA, and said endosomolytic spermine constitutes between 40% and 90%, inclusive, of the spermine-containing molecules in said composition.
4. The composition of preferred embodiment 1, wherein said nucleic acid comprises DNA, and said targeting spermine constitutes between 10% and 60%, inclusive, of the spermine-containing molecules in said composition.
5. The composition of preferred embodiment 1, wherein said nucleic acid is RNA, and said endosomolytic spermine constitutes between 20% and 90%, inclusive, of the spennine-containing molecules in said composition.
6. The composition of preferred embodiment 1, wherein said nucleic acid is RNA, and said targeting pennine constitutes between 10% and 80%, inclusive, of the spermine- containing molecules in said composition.
7. The composition of preferred embodiment 1, wherein said targeting spermine constitutes between 30 and 40%, inclusive, of the spermine-containing molecules in said composition, and wherein said endosomolytic spermine constitutes between 60 and 70%, inclusive of the spermine-containing molecules in said composition.
8. The composition of preferred embodiment 7, wherein said targeting spermine constitutes 35% of the spermine-containing molecules in said composition, and wherein said endosomolytic spermine constitutes 65% of the spermine-containing molecules in said composition.
9. The composition of preferred embodiment 1, further comprising a spermine-containing molecule that does not contain a cholesterol, a fatty acid, or a ligand for a cell surface molecule.
10. The composition of preferred embodiment 1, wherein the ionic strength of said composition is equivalent to the ionic strength of a solution containing between 50 mM and 240 mM sodium, inclusive.
11. The composition of preferred embodiment 10, wherein the ionic strength of said composition is equivalent to the ionic strength of a solution containing between 125 mM and 175 mM sodium, inclusive.
12. The composition of preferred embodiment 1, wherein the pH of said composition is between 6 and 8, inclusive.
13. The composition of preferred embodiment 12, wherein the pH of said composition is between 6 and 7, inclusive.
14. The composition of preferred embodiment 13, wherein the pH of said composition is between 6.5 and 6.8, inclusive.
15. The composition of preferred embodiment 1, wherein said nucleic acid is a DNA, RNA, DNA/RNA hybrid, or peptide nucleic acid.
16. The composition of preferred embodiment 1, wherein said nucleic acid is linear.
17. The composition of preferred embodiment 1, wherein said nucleic acid is circular.
18. The composition of preferred embodiment 1, wherein said nucleic acid is supercoiled.
19. The composition of preferred embodiment 1, wherein said nucleic acid is single stranded.
20. The composition of preferred embodiment 1, wherein said nucleic acid is double stranded.
21. The composition of preferred embodiment 1, wherein the length of said nucleic acid is less than 45 kilobases.
22. The composition of preferred embodiment 21, wherein the length of said nucleic acid is less than 10 kilobases.
23. The composition of preferred embodiment 22, wherein the length of said nucleic acid is less than 100 bases.
24. The composition of preferred embodiment 21, containing between 1 and 50 µg of nucleic acid.
25. The composition of preferred embodiment 1, wherein one said endosomolytic spermine comprises two cholesterols, two fatty acids, or one cholesterol and one fatty acid.
26. The composition of preferred embodiment 25, wherein said fatty acids are the same.
27. The composition of preferred embodiment 25, wherein said fatty acids are different.
28. The composition of preferred embodiment 1, wherein said targeting spermine comprises two ligands for a cell surface molecule.
29. The composition of preferred embodiment 28, wherein said ligands are the same.
30. The composition of preferred embodiment 24, wherein said ligands are different.
31. The composition of preferred embodiment 1, comprising at least two different endosomolytic spermines and/or at least two different targeting spermines.
32. The composition of preferred embodiment 31, wherein one endosomolytic spermine comprises a cholesterol and one endosomolytic spermine comprises a fatty acid.
33. The composition of preferred embodiment 32, comprising more cholesterol moieties than fatty acid moieties.
34. The composition of preferred embodiment 1, wherein said ligand is a peptide, antibody, biotin, a folate receptor ligand, lactose, fucose, or mannose moiety.
35. The composition of preferred embodiment 34, wherein said peptide comprises at most 10 amino acids, or said peptide comprises an RGD motif.
36. The composition of preferred embodiment 1, wherein said ligand is bound to a secondary amine in a spermine through a linker and/or an oxygen at the C3 position in said cholesterol is bound to a secondary amine in a spermine through a linker,
37. The composition of preferred embodiment 1, wherein said fatty acid is bonded directly to a secondary amine in a spermine and has a free COOH group.
38. The composition of preferred embodiment 36, wherein said linker contains between 3 and 12 carbon atoms, inclusive.
39. The composition of preferred embodiment 38, wherein said linker is a saturated or unsaturated C3 to C12 hydrocarbon moiety, inclusive.
40. The composition of preferred embodiment 38, wherein said linker contains 3 or 4 carbon atoms and no double bonds.
41. The composition of preferred embodiment 38, wherein said linker contains 5 or 6 carbon atoms and at most 1 double bond.
42. The composition of preferred embodiment 38, wherein said linker contains between 7 or 12 carbon atoms, inclusive, and at most 2 double bonds.
43. The composition of preferred embodiment 38, wherein said linker contains 5 carbon atoms.
44. The composition of preferred embodiment 38, wherein said linker is bound through a terminal carboxyl, amino, hydroxyl, sulfhydryl, alkyl, carboxamide, carbamate, thiocarbamate, or carbamoyl bridging group to a secondary amine group of the pennine.
45. The composition of preferred embodiment 1, wherein said fatty acid contains between 4 or 12 carbon atoms, inclusive.
46. The composition of preferred embodiment 45, wherein said linker is a saturated or unsaturated C4 to C12 hydrocarbon moiety, inclusive.
47. The composition of preferred embodiment 45, wherein said fatty acid comprises an ester group.
48. The composition of preferred embodiment 45, wherein said fatty acid contains 6 carbon atoms.
49. The composition of preferred embodiment 45, wherein the pKa of the carboxyl group of said fatty acid is at most 6.
50. A pharmaceutical composition comprising a composition of any one of preferred embodiments 1-49 and a pharmaceutically acceptable carrier.
51. The composition of preferred embodiment 50, wherein the pH of said composition is between 5 and 8, inclusive.
52. The composition of preferred embodiment 51, wherein the pH of said composition is between 6 and 7.5, inclusive.
53. The composition of preferred embodiment 50, wherein said composition is isotonic relative to the electrolyte concentration of human blood.
54. The composition of preferred embodiment 50, comprising between 1 and 30 µg nucleic acid.
55. A composition comprising a nucleic acid complexed with a cationic amphiphiles in an oil-in-water emulsion in which at least 10% of said complex is in the oil phase of said emulsion.
56. The composition of preferred embodiment 55, wherein at least 25% of said nucleic acid is in the oil phase of said emulsion.
57. The composition of preferred embodiment 56, wherein at least 50% of said nucleic acid is in the oil phase of said emulsion.
58. The composition of preferred embodiment 57, wherein at least 75% of said nucleic acid is in the oil phase of said emulsion.
59. The composition of preferred embodiment 58, wherein at least 90% of said nucleic acid is in the oil phase of said emulsion.
60. The composition of preferred embodiment 55, wherein said cationic amphiphile is a cationic lipid, modified or unmodified spermine, bupivacaine, or benzalkonium chloride.
61. The composition of preferred embodiment 55, wherein cationic amphiphile is (i) bupivacaine and the ratio of positive to negative charge is between 1 and 5, inclusive; (ii) unmodified or modified spermine the ratio of positive to negative charge is between 0.5 to 1.5, inclusive; (iii) a cationic lipid and the ratio of positive to negative charge is between 0.5 and 5.0, inclusive, (iv) lipofectamine and the ratio of positive to negative charge is between 0.5 and 2.0, inclusive; or (v) benzalkonium chloride and the ratio of positive to negative charge is between is 0.01 and 0.2, inclusive.
62. The composition of preferred embodiment 55, wherein said oil is a vegetable or animal oil.
63. The composition of preferred embodiment 55, wherein the pH of said composition is between 6 and 8, inclusive.
64. The composition of preferred embodiment 63, wherein the pH of said composition is between 6 and 7, inclusive.
65. The composition of preferred embodiment 64, wherein the pH of said composition is between 6.5 and 6.8, inclusive.
66. The composition of preferred embodiment 55, wherein said nucleic acid is a DNA, RNA, DNA/RNA hybrid, or peptide nucleic acid.
67. The composition of preferred embodiment 55, wherein said nucleic acid is linear.
68. The composition of preferred embodiment 55, wherein said nucleic acid is circular.
69. The composition of preferred embodiment 55, wherein said nucleic acid is supercoiled.
70. The composition of preferred embodiment 55, wherein said nucleic acid is single stranded.
71. The composition of preferred embodiment 55, comprising between 1 and 50 ug nucleic acid.
72. A pharmaceutical composition comprising a composition of any one of preferred embodiments 55-71 and a pharmaceutically acceptable carrier.
73. The composition of preferred embodiment 72, wherein the pH of said composition is between 5 and 8, inclusive.
74. The composition of preferred embodiment 73, wherein the pH of said composition is between 6 and 7.5, inclusive.
75. The composition of preferred embodiment 72, wherein said composition is isotonic relative to the electrolyte concentration of human blood.
76. The composition of preferred embodiment 72, comprising between 1 and 30 µg nucleic acid.
77. A method for expressing an RNA or protein molecule of interest in a cell, said method comprising contacting a cell with a composition of preferred embodiment 1,50, 55, or 72 under conditions that allow expression of an RNA or protein of interest encoded by a nucleic acid in said composition.
78. The method of preferred embodiment 77, wherein said cell has a mutation associated with a disease or disorder in an endogenous form of said RNA or protein of interest and said nucleic acid encodes a form of said RNA or protein that is not associated with said disease or disorder.
79. The method of preferred embodiment 77, wherein said RNA or protein of interest is from a pathogen, and said method causes an immune response against said RNA or protein of interest.
80. A method for inhibiting the expression of a target nucleic acid in a cell, said method comprising contacting a cell with a composition of preferred embodiment 1,50, 55, or 72 under conditions that allow expression of a ribozyme encoded by a nucleic acid in said composition, wherein said ribozyme cleaves a target nucleic acid in said cell that is associated with a disease, disorder, or infection.
81. A method for inhibiting the expression of a target nucleic acid in a cell, said method comprising contacting a cell with a composition of preferred embodiment 1,50, 55, or 72, wherein said composition comprises a first double stranded RNA (dsRNA) or a nucleic acid encoding a first double stranded dsRNA that has substantial sequence identity to a region of said target nucleic acid and specifically inhibits expression of said target nucleic acid.
82. The method of preferred embodiment 81, further comprising introducing a short, second dsRNA or a nucleic acid encoding a short, second dsRNA into said cell, wherein said second dsRNA inhibits dsRNA-mediated toxicity.
83. A method for treating, stabilizing, or preventing a disease, disorder, or infection in an animal, said method comprising contacting an animal with a composition of preferred embodiment 1,50, 55, or 72, wherein said composition comprises a first dsRNA or a nucleic acid encoding a first double stranded dsRNA that has substantial sequence identity to a region of said target nucleic acid associated with said disease, disorder, or infection and specifically inhibits expression of said target nucleic acid.
84. The method of preferred embodiment 83, further comprising introducing a short, second dsRNA or a nucleic acid encoding a short, second dsRNA into said cell, wherein said second dsRNA inhibits dsRNA-mediated toxicity.
85. A method for inhibiting the expression of a target nucleic acid in a cell, said method comprising contacting a cell with a composition of preferred embodiment 1,50, 55, or 72, wherein said composition comprises an antisense nucleic acid that has substantial sequence identity to a region of said target nucleic acid and specifically inhibits expression of said target nucleic acid.
86. A method for treating, stabilizing, or preventing a disease, disorder, or infection in an animal, said method comprising contacting an animal with a composition of preferred embodiment 1, 50, 55, or 72, wherein said composition comprises an antisense nucleic acid that has substantial sequence identity to a region of said target nucleic acid associated with said disease, disorder, or infection and specifically inhibits expression of said target nucleic acid.
87. The method of preferred embodiment 83 or 86, wherein said target nucleic acid is associated with a pathogen.
88. The method of preferred embodiment 87, wherein said pathogen is a virus, bacterium, yeast, or infectious agent.
89. The method of preferred embodiment 82 or 84, wherein the double stranded region in said second dsRNA contains between 11 and 30 nucleotides, inclusive.
90. The method of preferred embodiment 81 or 83, wherein the double stranded region in said first dsRNA contains between 11 and 30 nucleotides, inclusive.
91. The method of preferred embodiment 81 or 83, wherein the double stranded region in said first dsRNA comprises over 30 nucleotides.
92. The method of preferred embodiment 91, wherein the double stranded region in said first dsRNA comprises over 200 nucleotides.
93. The method of preferred embodiment 77,80, 81,83, 85, or 86, wherein said cell is a vertebrate cell.
94. The method of preferred embodiment 93, wherein said cell is a mammalian cell.
95. The method of preferred embodiment 94, wherein said cell is a human cell.
96. The method of preferred embodiment 77, 80, 81, 83, 85, or 86, wherein said cell is in a mammal.
97. The method of preferred embodiment 96, wherein said cell is in a human.
98. The method of preferred embodiment 83 or 86, wherein said animal is a mammal.
99. The method of preferred embodiment 98, wherein said animal is a human.

## Claims

1. A composition comprising a nucleic acid complexed with a cationic amphiphile in an oil-in-water (o/w) emulsion in which at least 10% of the nucleic acid - cationic amphiphile complex is in the oil phase of the oil-in-water emulsion.

2. The composition of claim 1, wherein the cationic amphiphile is a cationic lipid, modified or unmodified spermine, bupivacaine, or benzalkonium chloride.

3. The composition of any of claims 1-2, wherein the oil is a vegetable or an animal oil.

4. The composition of any of claims 1-3, wherein the composition has a pH of between 6 and 8, inclusive.

5. The composition of any of claims 1-4, wherein the nucleic acid is DNA, RNA, DNA/RNA hybrid, or peptide nucleic acid (PNA).

6. The composition of any of claims 1-5, wherein the nucleic acid is single-stranded or double-stranded.

7. The composition of any of claims 1-6, wherein the nucleic acid is linear, circular, or supercoiled.

8. The composition of any of claims 1-7, wherein the nucleic acid has a double-stranded region of between 11 and 45 contiguous nucleotides, inclusive.

9. The composition of any of claims 1-8, comprising between 1 and 50 µg of the nucleic acid.

10. The composition of any of claims 1-9, wherein the cationic amphiphile is (i) bupivacaine and the complex has a ratio of positive to negative charge of between 1 and 5, inclusive; (ii) unmodified or modified spermine and the complex has a ratio of positive to negative charge of between 0.5 to 1.5, inclusive; (iii) a cationic lipid and the complex has a ratio of positive to negative charge of between 0.5 and 5.0, inclusive, (iv) lipofectamine and the complex has a ratio of positive to negative charge of between 0.5 and 2.0, inclusive; or (v) benzalkonium chloride and the complex has a ratio of positive to negative charge ratio of between 0.01 and 0.2, inclusive.

11. A pharmaceutical composition comprising a composition of any one of claims 1-10 and a pharmaceutically acceptable carrier.

12. A method for expressing an RNA or protein molecule of interest in a cell, the method comprising contacting a cell with a composition of any of claims 1-11 under conditions that allow expression of an RNA or protein of interest encoded by a nucleic acid in the composition.

13. A method for inhibiting the expression of a target nucleic acid in a cell, the method comprising contacting a cell with a composition of any of claims 1-11 under conditions that allow expression of a ribozyme encoded by a nucleic acid in the composition, wherein the ribozyme cleaves a target nucleic acid in the cell.

14. A method for inhibiting the expression of a target nucleic acid in a cell, said method comprising contacting a cell with a composition of any of claims 1-11, wherein the composition comprises a first double stranded RNA (dsRNA) or a nucleic acid encoding a first double stranded dsRNA, wherein the first dsRNA has substantial sequence identity to a region of a target nucleic acid and specifically inhibits expression of the target nucleic acid.

15. A method for inhibiting the expression of a target nucleic acid in a cell, the method comprising contacting a cell with a composition of any of claims 1-11, wherein the composition comprises an antisense nucleic acid that has substantial sequence identity to a region of a target nucleic acid and specifically inhibits expression of the target nucleic acid.
